(19)

Europäisches
Patentamt
European
Patent Office
Office européen
des brevets

(11) **EP 1 752 899 A1**

(12) **EUROPEAN PATENT APPLICATION**

(43) Date of publication:
**14.02.2007 Bulletin 2007/07**

(51) Int Cl.:
***G06F 19/00*** (2006.01)

(21) Application number: **06016619.6**

(22) Date of filing: **09.08.2006**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(30) Priority: **09.08.2005 JP 2005231005**

(71) Applicant: **Ajinomoto Co., Inc.
Tokyo 104-8315 (JP)**

(72) Inventors:
• **Usuda, Yoshihiro
Kawasaki-shi
Kanagawa 210-8681 (JP)**

• **Iwatani, Shintaro
Kawasaki-shi
Kanagawa 210-8681 (JP)**
• **Matsui, Kazuhiko
Kawasaki-shi
Kanagawa 210-8681 (JP)**

(74) Representative: **HOFFMANN EITLE
Patent- und Rechtsanwälte
Arabellastrasse 4
81925 München (DE)**

(54) **Method for simulating a production process of a substance**

(57)     A method for simulating a substance production process using cells based on differential equations concerning intracellular metabolites and gene expression comprising preparing the differential equations concerning intracellular metabolites and gene expression, assigning values for parameters in the differential equations required for the simulation and solving the differential equations with the assigned values, which is characterized by the following (a) to (d): (a) the differential equations include a differential equation including a specific growth rate of the cells, and the specific growth rate is represented as a function of time; (b) all or a part of parameters of the differential equations are represented as a function of the specific growth rate or a function of time; (c) the differential equations include a formation rate for formation of a cell component from an intracellular metabolite, and the formation rate is represented as a function of the specific growth rate or a function of time; and (d) the differential equations include an inflow rate of a metabolite taken up from the outside of the cells and/or an outflow rate of a metabolite excreted out of the cells from the inside of the cells, and the inflow rate and/or the outflow rate is represented as a function of the specific growth rate or a function of time.

**EP 1 752 899 A1**

**Description**

BACKGROUND OF THE INVENTION

The present invention relates to a method for simulating a production process of a substance, typically an amino acid or a nucleic acid, using cells of a microorganism or the like, and a program therefor.

The technique of constructing a mathematical equation model of biochemical reactions caused by intracellular enzymes to estimate intracellular dynamic behaviors of metabolites is called metabolic simulation, and there are many examples thereof (Ishii, N. et al., J. Biotechnol., 113:281-294, 2004) and proposed many methods therefor (U.S. Patent Application No. 2002/0022947, International Publication Nos. WO2004/081862, WO03/07217, WO02/55995, WO02/05205, Japanese Patent Application Laid-Open (KOKAI) No. 2003-180400). As an example of comparatively large scale metabolic simulation, Teusink et al. performed simulation of anaerobic ethanol fermentation of *Saccharomyces cerevisiae* considering metabolic routes branching from the glycolytic system for producing glycogen, trehalose, glycerol and succinic acid (Teusink, B. et al., Eur. J. Biochem., 267:5313-5329, 2000). For *Escherichia coli,* Chassagnole et al. constructs a central metabolic model under a condition of a constant growth rate to perform simulation (Chassagnole, C. et al., Biotechnol. Bioeng., 26:203-216, 2002). According to another report, a part of the gene expression of *E. coli* metabolic enzymes was modeled and combined with an enzymatic reaction model to perform simulation (Wang, J. et al., J. Biotechnol., 92:133-158, 2001; Schmid J.W. et al., Metab. Eng., 6:364-377, 2004). Further, in the report of Varner, construction of a large scale model in which gene expression is incorporated into a kinetic model of enzymes is conceptually disclosed, and a specific growth rate is expressed by an equation using saturation coefficients of precursors for the maximum specific growth rate (Varner, J.D., Biotechnol. Bioeng., 69:664-678, 2000). For other organisms, further detailed models have been reported. Jeong et al. constructed a model of sporulation process of *Bacillus subtilis* in batch culture using mathematical equations (Jeong et al., Biotechnol. Bioeng., 35:160-184, 1990). Tomita et al., Bioinformatics 15:72-84, 1999, also has reported on the simulation of 127 genes involved in transcription and translation of the *Mycoplasma genitalium* genome, as well as energy production and phospholipid synthesis of the microorganism.

SUMMARY OF THE INVENTION

In simulation of a production process of a substance, typically a nucleic acid or an amino acid, using cells of microorganisms or the like, enzymatic reactions from a substrate to an objective product are represented by mathematical equations using kinetic parameters in many cases. However, batch culture or semi-batch culture (fed batch culture) is often used for production of a useful substance, and therefore the growth rate of cells changes. In connection with it, various kinds of parameters in the cells also change. In addition, besides the production rates of a substance serving as a substrate, components present in the medium and an objective product, production rates of by-products such as amino acids, organic acids and carbon dioxide ($CO_2$) also change during the process of substance production. Therefore, a technique for performing metabolic simulation with sufficient precision in such a manner that the simulation should well fit to experimental data of such growth rates or by-products is desired. If an accurate metabolic simulation reflecting experimental data is enabled, it becomes possible to conduct experiments of amplification or deletion of a gene by a computer in a short time (in silico experiments). It is expected that it should greatly shorten the development period for improving substance production ability of cells.

The inventors of the present invention conducted various experiments in view of the aforementioned problems. Consequently, they found that they could achieve more accurate metabolic simulation of the production of a substance, typically an amino acid or a nucleic acid, by cells of microorganisms or the like. The enhanced accuracy pertained when (A) a specific growth rate, serving as an index of cell growth, was represented by a mathematical equation that used a time function based on measured values, and (B) time functions or functions using the specific growth rate as a variable were employed with various parameters, including outflow rates of intracellular metabolites into cell components and, further, uptake rates of intracellular metabolites from the outside of the cells or excretion rates of the same to the outside of the cells.

The present invention was accomplished based on the aforementioned findings and provides the following.

[1] A method for effecting a simulation of a substance-production process that uses cells, wherein said simulation is based on a set of differential equations that represent intracellular metabolites and gene expression, said method comprising the steps of:

    (a) including a specific growth rate of cells, expressed as a differential equation, in the set of differential equations;
    (b) assigning values for parameters in the set of differential equations, wherein at least one of said parameters is represented as a growth rate factor;
    (c) incorporating in the set of differential equations a formation rate for formation of a cell component from an intracellular metabolite, wherein said formation rate is represented as a growth rate factor;
(d) incorporating in the set of differential equations an inflow rate of a metabolite taken up from the outside of the cells and/or an outflow rate of a metabolite excreted out of the cells from the inside of the cells, wherein said inflow rate and said outflow rate are represented, respectively, as a growth rate factor;
(e) solving the set of differential equations; and
(f) generating data representative of the substance-production process.

[2] The method according to [1], wherein the differential equations including the specific growth rate of the cells include the differential equations represented as the following equations (1) to (3):

$$d[Metabolite]/dt = V_{input} - V_{output} - \mu[Metabolite] \quad (Equation\ 1)$$

$$d[mRNA]/dt = k_{transcription}[P] - (k_{dRNA} + \mu)[mRNA] \quad (Equation\ 2)$$

$$d[Protein]/dt = k_{translation}[mRNA] - (k_{dProtein} + \mu)[Protein] \quad (Equation\ 3)$$

wherein, in the equation 1, [Metabolite] represents an intracellular concentration of a metabolite, $V_{input}$ represents the sum of rates of reactions producing the metabolite, $V_{output}$ represents the sum of rates of reactions consuming the metabolite, and $\mu$ represents the specific growth rate;
in the equation 2, [mRNA] represents a concentration of mRNA, $k_{transcription}$ represents a rate constant of transcription, [P] represents a promoter concentration, $k_{dRNA}$ represents a rate constant of decomposition of mRNA, and $\mu$ represents the specific growth rate, and
in the equation 3, [Protein] represents a concentration of a protein, $k_{translation}$ represents a rate constant of translation, $k_{dProtein}$ represents a rate constant of decomposition of the protein, and $\mu$ represents the specific growth rate.
[3] The method according to [1], wherein the growth rate factor is a function of the specific growth rate or a function of time.
[4] The method according to [1], wherein the specific growth rate is represented as a function of time, and the function is obtained by preparing a mathematic equation from measurement data of the specific growth rate in the production process.
[5] The method according to [1], wherein the growth rate factor representing the formation rate is obtained by preparing a mathematic equation expressing measurement data of the formation rate in the production process.
[6] The method according to [1], wherein the growth rate factor representing the inflow rate and/or the outflow rate is obtained by preparing a mathematic equation expressing measurement data of the inflow rate and/or the outflow rate in the production process.
[7] The method according to [1], wherein the metabolite taken up into the cells is a substrate and/or an organic substance in a medium.
[8] The method according to [1], wherein the metabolite excreted out of the cells is an objective substance and/or a by-product.
[9] The method according to [8], wherein the metabolite excreted out of the cells is an amino acid, an organic acid and/or carbon dioxide.
[10] The method according to [9], wherein the metabolite excreted out of the cells is an amino acid or an organic acid.
[11] The method according to [1], wherein the parameters required for the simulation are a rate constant of transcription and/or a rate constant of translation.
[12] The method according to [1], wherein the cells are those of a microorganism having an amino acid producing ability and/or an organic acid producing ability.
[13] The method according to [12], wherein the microorganism is *Escherichia coli*.
[14] The method according to [1], wherein a composition of cell components itself is represented by a mathematical equation using the specific growth rate of the cells or the cells' equivalent index concerning the growth.
[15] A computer program product for effecting a simulation of a substance-production process that uses cells, wherein said simulation is based on a set of differential equations that represent intracellular metabolites and gene expression, comprising:

> (a) computer code for including a specific growth rate of cells, expressed as a differential equation, in the set of differential equations;
> (b) computer code for assigning values for parameters in the set of differential equations, wherein at least one of said parameters is represented as a growth rate factor;
> (c) computer code for incorporating in the set of differential equations a formation rate for formation of a cell component from an intracellular metabolite, wherein said formation rate is represented as a growth rate factor;
> (d) computer code for incorporating in the set of differential equations an inflow rate of a metabolite taken up from the outside of the cells and/or an outflow rate of a metabolite excreted out of the cells from the inside of the cells, wherein said inflow rate and said outflow rate are represented, respectively, as a growth rate factor;
> (e) computer code for solving the set of differential equations; and
> (f) computer code for generating data representative of the substance-production process.

[16] A system for effecting a simulation of a substance-production process that uses cells, wherein said simulation is based on a set of differential equations that represent intracellular metabolites and gene expression, comprising:
a processor for processing information; and
a storing means, including:

> (a) computer code for including a specific growth rate of cells, expressed as a differential equation, in the set of differential equations;
> (b) computer code for assigning values for parameters in the set of differential equations, wherein at least one of said parameters is represented as a growth rate factor;
> (c) computer code for incorporating in the set of differential equations a formation rate for formation of a cell component from an intracellular metabolite, wherein said formation rate is represented as a growth rate factor;
> (d) computer code for incorporating in the set of differential equations an inflow rate of a metabolite taken up from the outside of the cells and/or an outflow rate of a metabolite excreted out of the cells from the inside of the cells, wherein said inflow rate and said outflow rate are represented, respectively, as a growth rate factor;
> (e) computer code for solving the set of differential equations; and
> (f) computer code for generating data representative of the substance-production process.

According to the method of the present invention, it becomes possible to perform simulation of a production process of a substance, typically an amino acid or a nucleic acid, for a production process using cells of a microorganism or the like, in which a growth rate markedly changes.

BRIEF EXPLANATION OF DRAWINGS

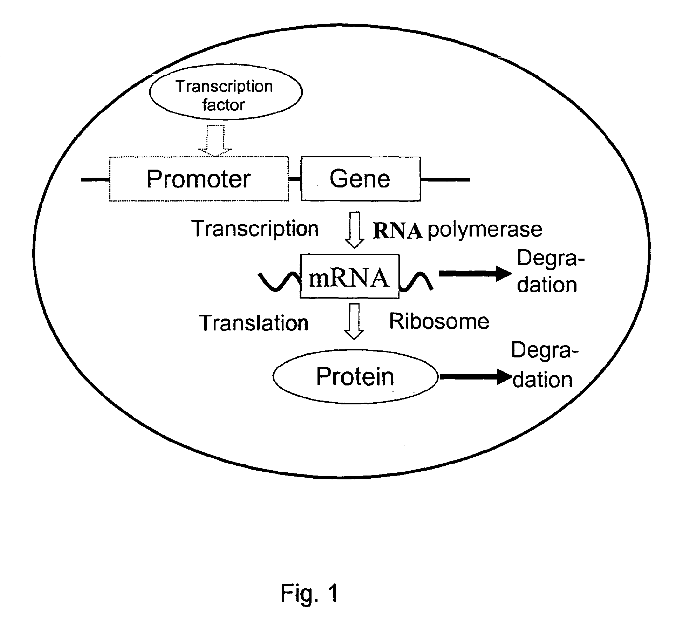

Fig. 1 shows a diagram for explaining modeling of gene expression.

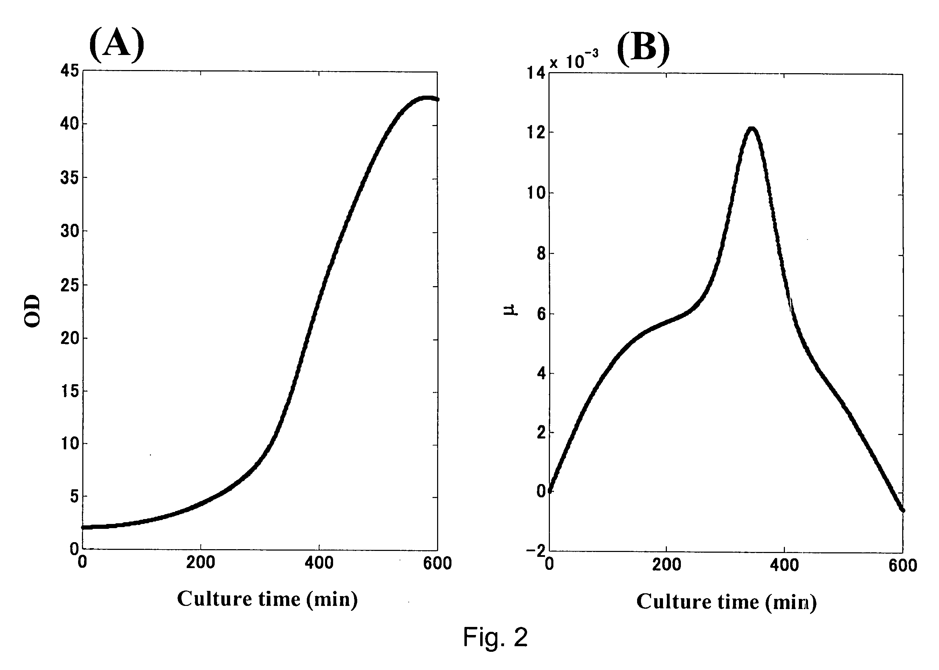

Fig. 2 shows examples of a growth curve obtained by measurement of turbidity (OD) (A) and a time equation of a specific growth rate $\mu$ obtained from the curve (B).

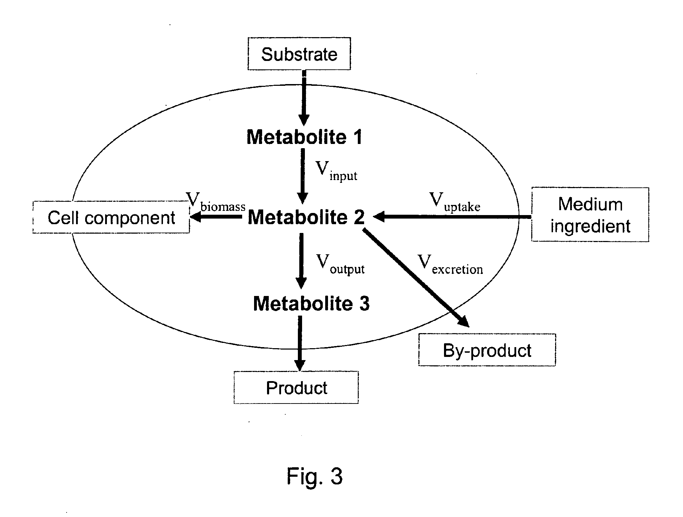

Fig. 3 shows a diagram for explaining the material balance of metabolites.

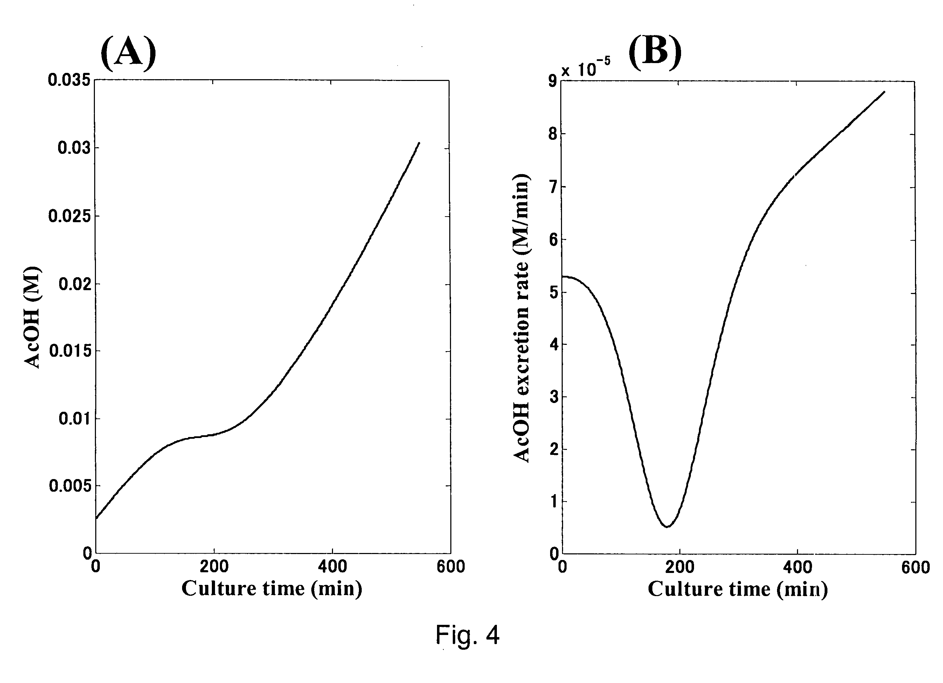

Fig. 4 shows examples of a concentration change curve obtained by measurement of an extracellular acetic acid (AcOH) concentration (A) and a time equation of an excretion rate obtained from the curve (B).

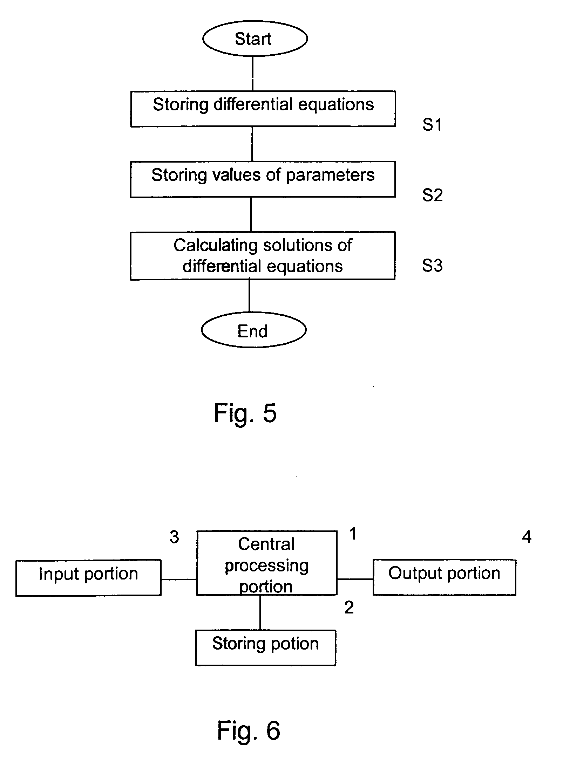

Fig. 5 shows a flowchart of a process to be executed by the program of the present invention.
Fig. 6 shows a functional block diagram of a computer on which the program of the present invention is installed.

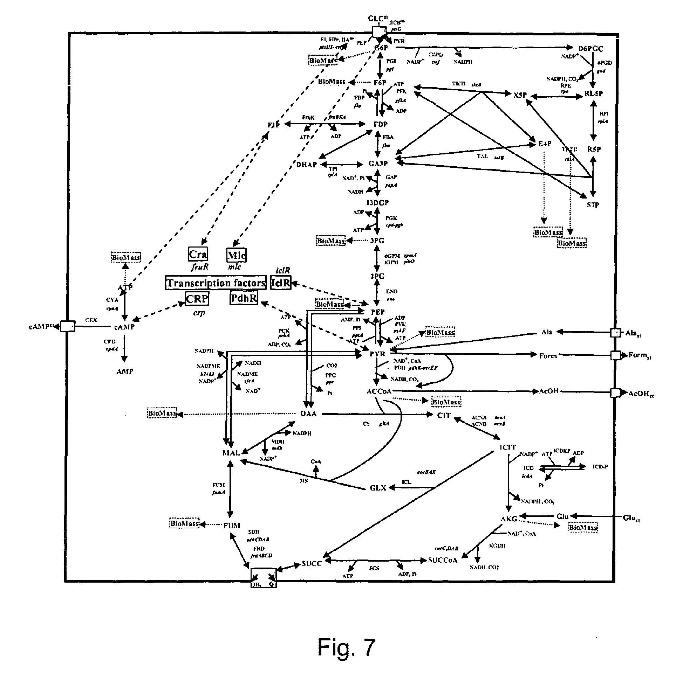

Fig. 7 shows a modeled central metabolic map of E. coli. Metabolites, enzymes and genes are indicated with abbreviations. The arrows represent conversion between metabolites by enzymatic reactions, for each of which name of enzyme (capital letters) and gene encoding the enzyme (small letters in italics) are indicated. The proteins in the boxes are transcription factors, and the broken lines represent interactions with an effector. The dotted lines represent outflow from intracellular metabolites to cell components. The thick black frame represents the cell membrane, the substances outside the cells are extracellular substances, and the boxes on the cell membrane indicate uptake or excretion.

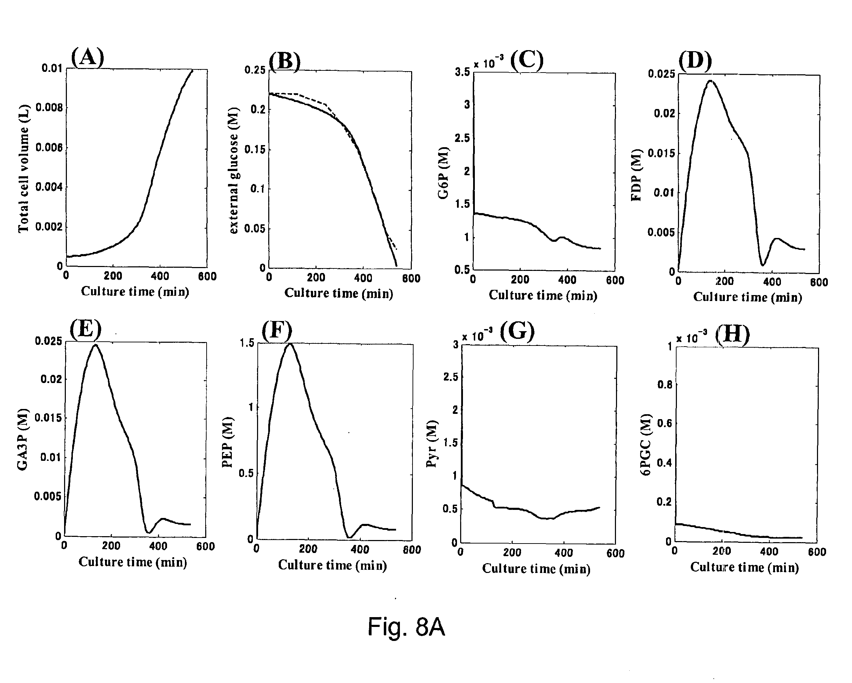

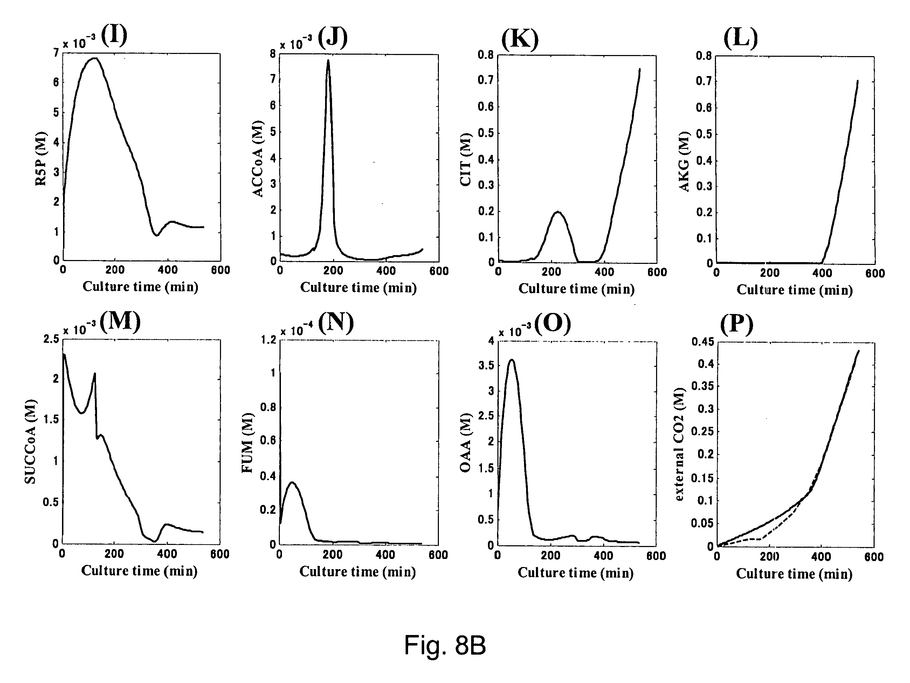

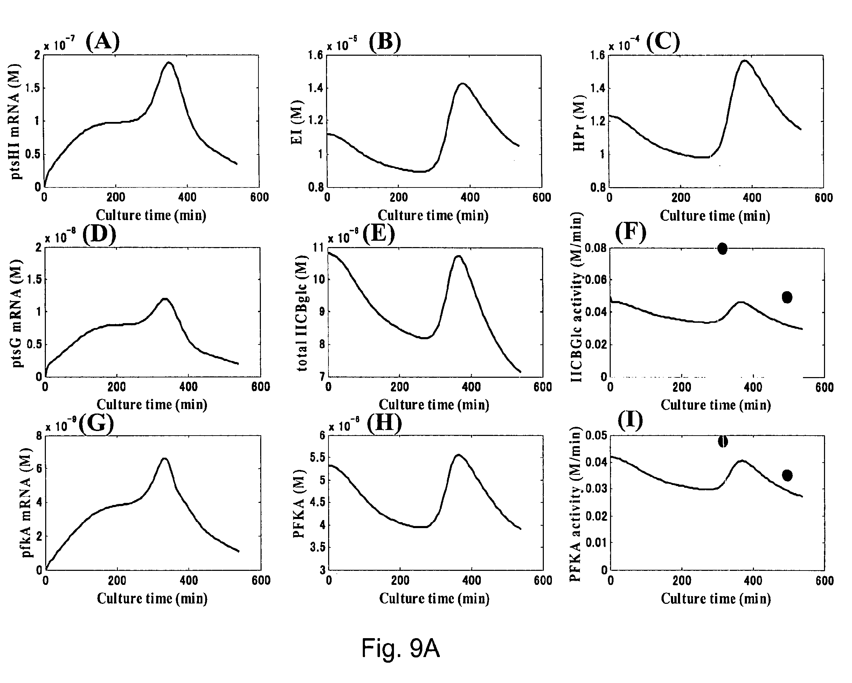

Figs. 8A and 8B show results of metabolic simulation of the E. *coli* central metabolic model: cell volume (growth) (A), extracellular glucose (B), G6P: (C), FDP (D), GA3P (E), PEP (F), PYR (G), 6PGC (H), R5P (I), ACCoA (J), CIT (K), AKG (L), SUCCoA (M), FUM (N), OAA (0), and extracellular $CO_2$ (P). For the extracellular glucose (B) and extracellular $CO_2$ (P), actual measured values are plotted as broken lines. The horizontal axes represent the culture time (minute) (the same shall apply to Figs. 9A, 9B, 10A, 10B, 11A, 11B, 12A and 12B).

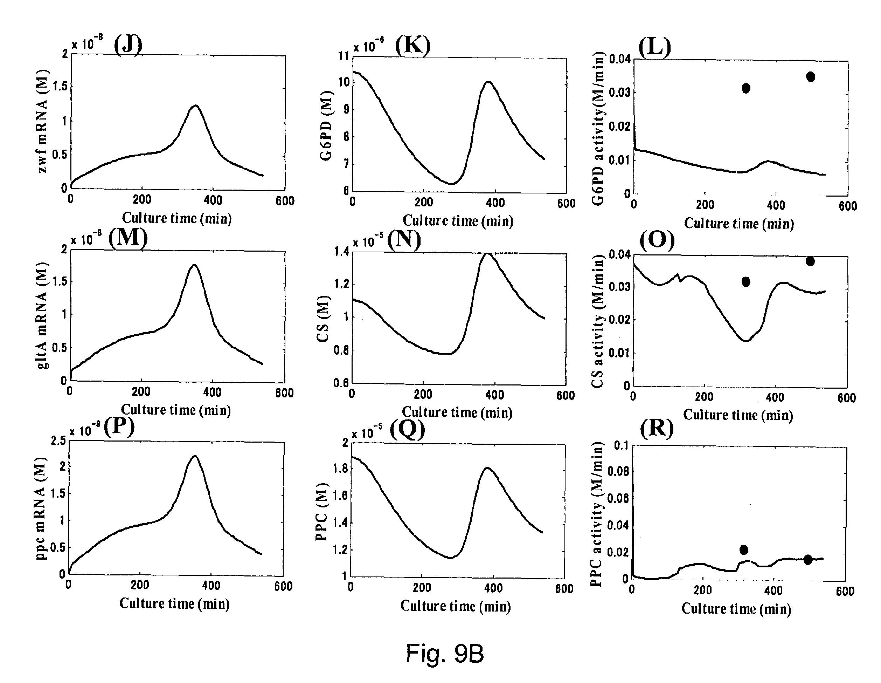

Figs. 9A and 9B show results of gene expression simulation of the E. *coli* central metabolic model: ptsHI mRNA (A), EI (B), HPr (C), ptsG mRNA (D), IICBGlc (E), IICBGlc activity (F), pfkA mRNA (G), PFKA (H), PFKA activity (I), zwf mRNA (J) G6PD (K), G6PD activity (L), gltA mRNA (M), CS (N), CS activity (0), ppc mRNA (P), PPC (Q), and PPC activity (R). The values of metabolic fluxes at 315 minutes and 495 minutes converted into enzymatic reaction rates are indicated with closed circles.

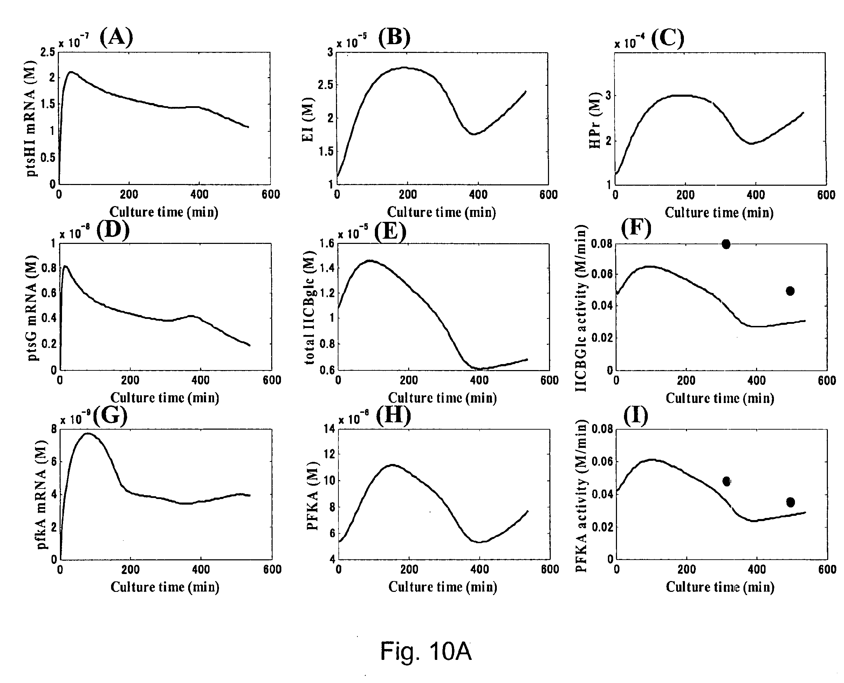

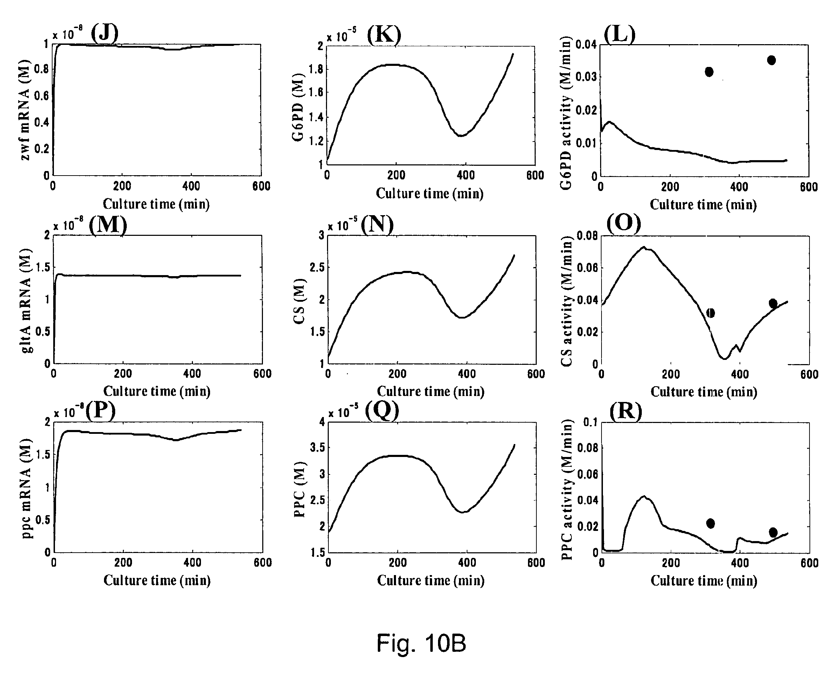

Figs. 10A and 10B show results of gene expression simulation under a condition that RNA polymerase and ribosome concentrations are independent from μ: ptsHI mRNA (A), EI (B), HPr (C), ptsG mRNA (D), IICBGlc (E), IICBGlc activity (F), pfkA mRNA (G), PFKA (H), PFKA activity (I), zwf mRNA (J), G6PD(K), G6PD activity (L), gltA mRNA (M), CS (N), CS activity (0), ppc mRNA (P), PPC (Q), and PPC activity (R). The values of metabolic fluxes at 315 minutes and 495 minutes converted into enzymatic reaction rates are indicated with closed circles.

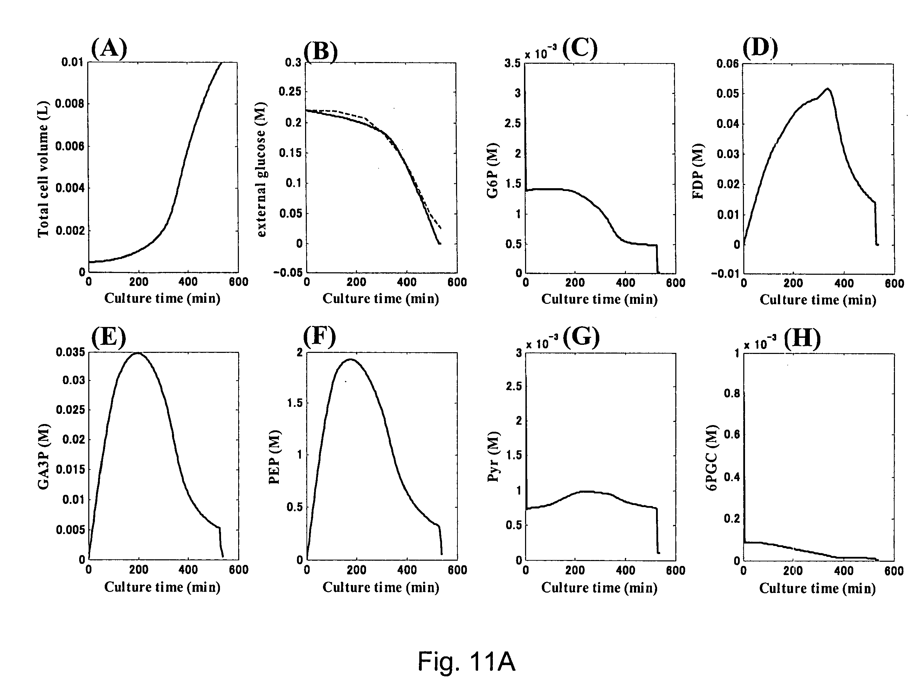

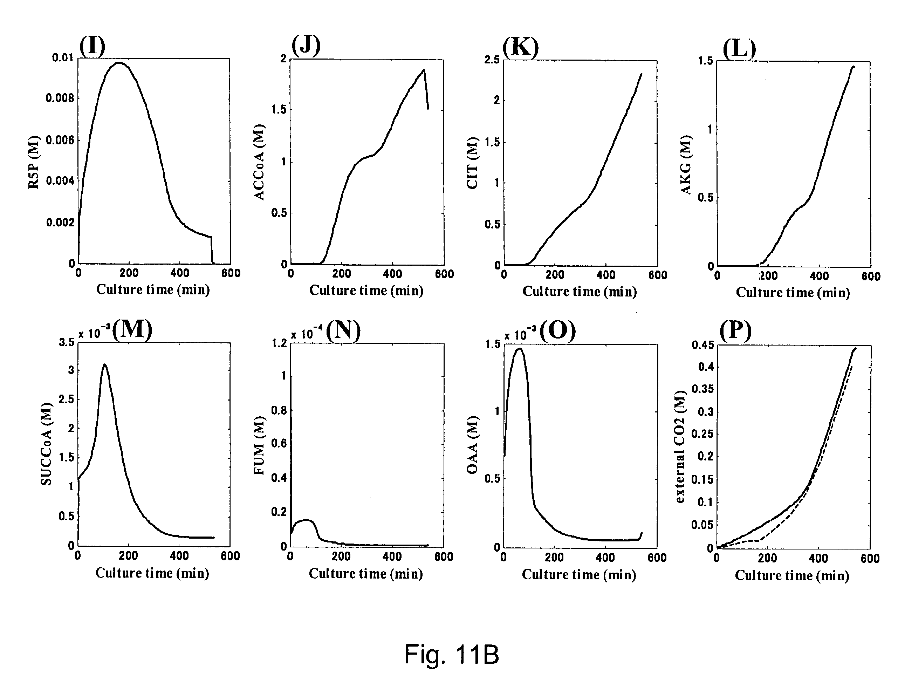

Figs. 11A and 11B show results of metabolic simulation when synthesis rates for cell components are set at 0: cell volume (growth) (A), extracellular glucose (B), G6P: (C), FDP (D), GA3P (E), PEP (F), PYR (G), 6PGC (H), R5P (I), ACCoA (J), CIT (K), AKG (L), SUCCoA (M), FUM (N), OAA (O), and extracellular $CO_2$ (P). For the extracellular glucose (B) and extracellular $CO_2$ (P), actual measured values are plotted as broken lines.

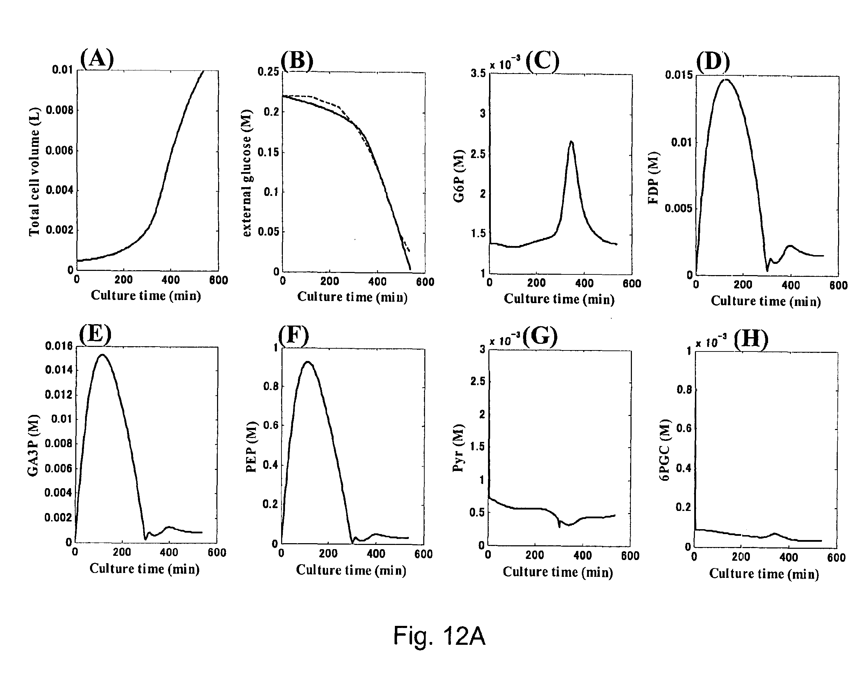

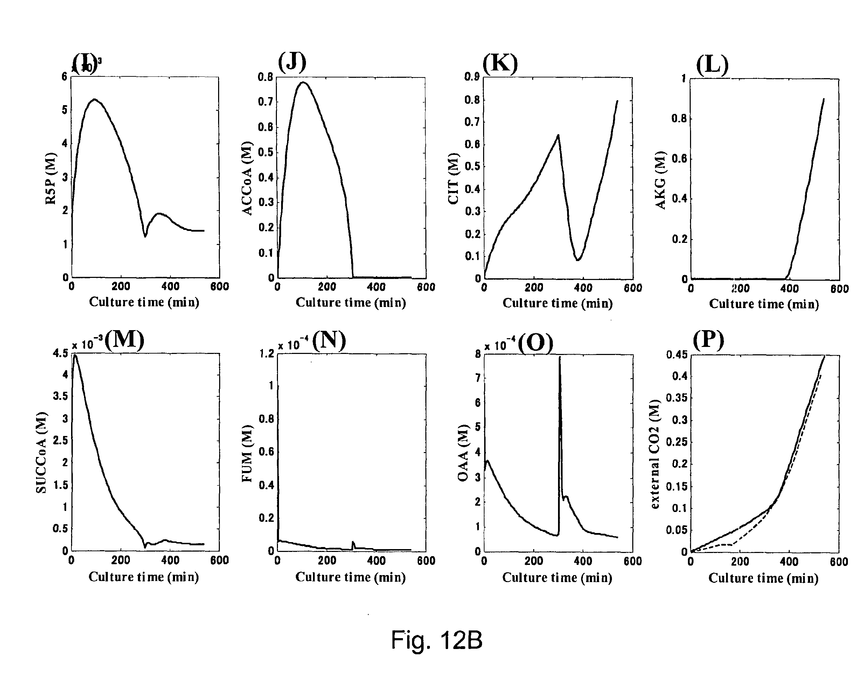

Figs. 12A and 12B show results of metabolic simulation when uptake and excretion of metabolites are set at 0: cell volume (growth) (A), extracellular glucose (B), G6P: (C), FDP (D), GA3P (E), PEP (F), PYR (G), 6PGC (H), R5P (I), ACCoA (J), CIT (K), AKG (L), SUCCoA (M), FUM (N), OAA (O), and extracellular $CO_2$ (P). For the extracellular glucose (B) and extracellular $CO_2$ (P), actual measured values are plotted as broken lines.

DETAILED DESCRIPTION OF THE INVENTION

Hereafter, the present invention will be explained in detail.

In this description, the phrase "substance production process using cells" and similar terminology refers to a process of biochemical reactions from a substrate to a product that is caused as sequential enzymatic reactions, using cells to produce an objective product.

The simulation method of the present invention is based on differential equations, which may be the same as a conventional simulation methodology except that specific conditions according to the present invention are employed. Conventional simulation methods comprise preparing differential equations for intracellular metabolites and gene expression, assigning values for parameters in the differential equations required for the simulation and solving the differential equations with the assigned values.

Differential equations can usually be prepared by incorporating mathematical equations for expression control into metabolic simulation.

Metabolic simulation is a technique for describing time-dependent dynamic changes by expressing intracellular biochemical reactions with mathematical equations, describing changes of substances caused by the reactions with differential equations, and solving them by numerical computation. The mathematical equations used for this purpose are often nonlinear ordinary differential equations (ODE), and this process of preparing mathematical equations is generally called "modeling." Typically, many nonlinear differential equations are solved by using a computer.

In this regard, the phrase "biochemical reactions" refers to processes for converting an intracellular metabolite by an enzymatic reaction. Data on such reactions are stored in databases for many organisms. For example, KEGG (Kyoto Encyclopedia of Genes and Genomes, http://www.genome.ad.jp/kegg/; Kanehisa, M. et al., Nucleic Acids Res., 32:277-280, 2004) can be referred to. As for E. coli, EcoCyc (Encyclopedia of *Escherichia coli* K12 Genes and Metabolism, http://ecocyc.org/, Keseler et al. (2005) Nucleic Acids Res., 33, D334-D337, 2005) is known. For describing these biochemical enzymatic reactions with mathematical equations, dynamic equations based on Michaelis-Menten type reaction formulas are often used (Segel I.H., Enzyme Kinetics: Behavior and Analysis of Rapid Equilibrium and Steady-State Enzyme Systems, John Wiley & Sons, 1975). Parameters of respective enzymes can be collected from literatures. For example, for E. *coli,* Chassagnole et al. described enzymatic reactions of the glycolytic pathway and pentose phosphate pathway from glucose to acetyl-CoA with mathematical equations using kinetic parameters (Chassagnole, C. et al., Biotechnol. Bioeng., 26, 203-216, 2002).

It is gene expression that determines amounts of intracellular enzymes, and this process is realized through the processes of transcription of a gene into mRNA and translation of mRNA into a protein. By incorporating this gene expression into metabolic simulation, it becomes possible to describe more detailed intracellular behaviors. Examples include description of expression control with mathematical equations and incorporation of them into metabolic simulation. For example, Wang et al. described expression control of the sucrose and glycerol uptake system of E. *coli* with mathematical equations and combined them with an enzymatic reaction model of the glycolytic pathway to perform simulation (Wang, J. et al., J. Biotechnol., 92:133-158, 2001), and Schmid et al. modeled expression control of a tryptophan biosynthesis pathway gene (trp operon) and combined it with a central metabolic model of *E. coli* to perform analysis (Schmid J.W. et al., Metab. Eng., 6:364-377, 2004). Differential equations concerning intracellular metabolites and gene expression are prepared as described above.

Concentration change and gene expression of a metabolite can be generally represented by one or more differential equations. Thus, one commonly represents each intracellular metabolite with an equation 1, considering the enzymatic reaction rate for each intracellular metabolite and the dilution effect due to growth:

$$\mathbf{d[Metabolite]/dt = V_{input} - V_{output} - \mu[Metabolite]} \quad \text{(equation 1)}$$

In the equation 1, [Metabolite] represents the concentration of an intracellular metabolite, $V_{input}$ represents the sum of rates of enzymatic reactions for producing the metabolite, $V_{output}$ represents the sum of rates of enzymatic reactions consuming the metabolite, and $\mu$ represents the specific growth rate.

Gene expression is generally described in terms of the two stages of transcription and translation, i.e., as changes in concentrations of mRNA produced by transcription of a gene and protein produced by translation of mRNA (Fig. 1).

For mRNA produced by transcription of various genes, one may describe the concentration by the following equation, considering the transcription rate with which mRNA is synthesized from a gene and the decomposition rate of mRNA as well as the dilution effect:

$$\mathbf{d[mRNA]/dt = k_{transcription}[RNAP][Promoter] - (k_{dmRNA} + \mu)[mRNA]}$$

$$\mathbf{(equation\ 2)}$$

In equation 2, [mRNA] represents the concentration of mRNA, $k_{transcription}$ represents a rate constant of transcription, [RNAP] represents the concentration of RNA polymerase which performs the transcription, [Promoter] represents the concentration of a promoter of the corresponding gene, $k_{dRNA}$ represents a rate constant of decomposition of mRNA, and $\mu$ represents the specific growth rate.

For a protein produced by translation of mRNA, the concentration can be described by the following equation, which takes into account the translation rate with which mRNA is translated into a protein and decomposition rate of the protein as well as the dilution effect:

$$\mathbf{d[Protein]/dt = k_{translation}[Ribosome][mRNA] - (k_{dProtein} +}$$

$$\mathbf{\mu)[Protein]} \quad \text{(equation 3)}$$

In equation 3, [Protein] represents the concentration of a protein, $k_{translation}$ represents a rate constant of translation, [Ribosome] represents the concentration of ribosome which performs translation, $k_{dProtein}$ represents a rate constant of protein decomposition, and $\mu$ represents the specific growth rate.

Equations 2 and 3 can also be described in various other ways.

When differential equations are prepared, values are assigned for the parameters required for simulation in the prepared differential equations. The parameters required for the simulation include rate constants, initial concentrations, and so forth. The parameters are preferably a rate constant of transcription and/or a rate constant of translation.

Parameters for respective enzymatic reactions and gene expression can be collected from literatures. However, there are many parameters for modeling of enzymatic reactions and gene expression, and therefore it is often impossible to collect all the parameters from literatures. In such a case, it is possible to estimate appropriate values from various information in literatures, or estimate them by optimization of measurement results obtained in experiments.

In order to solve such nonlinear ordinary differential equations obtained as described above, it is possible to use a mathematical calculation program such as MATLAB® (MathWorks) and MATHEMATICA® (Wolfram Research). To execute metabolic simulation, for example, ODE solvers of MATLAB® (MathWorks) can be used. As the ODE solver for solving a metabolic reaction equation or gene expression equation, ode45, ode21s and so forth are preferably used. Moreover, many kinds of metabolic simulation software have been developed as software for performing metabolic simulation, and they can be utilized (Ishii, N. et al., J. Biotechnol., 113:281-294, 2004). Examples include GEPASI (Mendes, P. Comput. Applic. Biosci., 9:563-571, 1993), SCAMP (Sauro, H.M., Comput. Appl. Biosci., 9:441-450, 1993), E-CELL (Tomita, M. et al., Bioinformatics, 15:72-84, 1999), and so forth.

A simulation of the present invention is characterized by additionally using certain conditions, as described in greater detail below. This approach enables more accurate simulation of a substance production process accompanied by growth of cells.

(a) Description of specific growth rate with time function

A differential equation that incorporates a specific growth rate of cells is included in the differential equations, and the specific growth rate is represented with a function of time.

The phrase "growth of cells" refers to a phenomenon whereby the number of cells increases in a substance production process. The number of cells usually increases with conversion of a substrate added for the substance production into cell components. When growth is represented by the number of cells, the growth rate is the rate at which the number of cells increases, and a specific growth rate is obtained by dividing the increase rate of the number of cells with the number of cells. The number of cells used in this context is one of the values serving as indexes of growth of cells, and any value may be used so long as a value having an equivalent function (for example, turbidity of culture broth) is chosen.

The function of time of the specific growth rate is preferably obtained by preparing mathematical equations that express measurement data of the specific growth rate in a production process. Growth of cells can be measured by measuring turbidity of culture broth or counting the number of cells in a diluted culture broth. A curve of cell growth experimentally measured can be approximated as a function of time. A large number of products are marketed as software for obtaining such an approximate mathematical equation, and preferred examples include TableCurve® 2D (Systat Software). The obtained time function of growth curve can be differentiated and divided with the equation of the growth curve to obtain a time equation of the specific growth rate. Examples of the growth curve obtained by measurement of turbidity (OD) and the time equation of the specific growth rate $\mu$ obtained therefrom are shown in Fig. 2.

(b) Representation of parameters with a growth rate factor

One or more of the parameters of the differential equations are represented as a growth rate factor.

The parameters change with the growth of cells. Thus, it is preferable to represent as many parameters as possible with a growth rate factor. The growth rate factor can be expressed as a function of time. A function of time of the specific growth rate is generated with measurement data relating to the specific growth rate obtained in a production process. In the alternative, the growth rate factor can be expressed as a function of the specific growth rate $\mu$. The specific growth rate $\mu$ may be obtained by dividing the rate of increase of the number of cells by the number of cells.

For example, it is known that rate constants of transcription and translation, which are parameters of gene expression, markedly change with the growth rate of cells, and molecular numbers of RNA polymerase and ribosome, which catalyze the respective processes, markedly change with the culture rate (Bremer and Dennis, In Escherichia coli and Salmonella: Cellular and Molecular Biology/Second Edition (Neidhardt, F.C. Ed.), pp. 1553-1569, American Society for Microbiology Press, Washington, D.C., 1996). If the rate constants of transcription and translation are expressed as specific growth rate-dependent mathematical equations and incorporated into mathematical equations for gene expression, it becomes possible to prepare mathematical equations that accurately describe the expression. More specifically, in the aforementioned differential equation of mRNA (equation 2), [RNAP] can be represented with an equation of specific growth rate $\mu$. Similarly, in the aforementioned differential equation of protein (equation 3), [Ribosome] can be represented with an equation of the specific growth rate $\mu$. The specific growth rate $\mu$ used herein may be the same as that used in (a) mentioned above, or a specific growth rate based on another index relating to growth of cells.

As mentioned above, it is also possible to directly express values measured in a cell growth process as a function of time. For example, if a function representing a parameter is represented with a specific growth rate-dependent equation, the parameter can be converted into a time function by substituting a time function of the specific growth rate into the specific growth rate-dependent equation.

(c) Description of cell component formation rate with a growth rate factor

A formation rate with which a cell component is formed from an intracellular metabolite is incorporated into the differential equations, and the formation rate is represented as a function of a growth rate factor. As set forth above, the growth rate factor can be expressed as a function of time or as a function of the specific growth rate $\mu$.

The phrase "cell component" refers to a major polymer compound constituting cells such as protein, RNA, DNA, lipid and lipopolysaccharide. By enzymatic conversion of a substrate into a cell component such as protein, nucleic acid and lipid, cells can obtain a required component. By enumerating biochemical reactions resulting in such a cell component, a stoichiometric matrix can be created (Savinell J.M., Palsson B.O., J. Theor. Biol., 154:421-454, 1992; Vallino, J.J. and Stephanopoulos, G., Biotechnol. Bioeng., 41:633-646, 1993). Details of creation of a stoichiometric matrix of metabolic reactions from glucose to all the amino acids in *E. coli* are described in WO2005/001736 in detail. If a composition of the cell components is given, the formation rates of the cell components from intracellular metabolites, $V_{biomass}$, can be calculated by using the stoichiometric matrix (Pramanik, J. and Keasling J.D., Biotechnol. Bioeng., 56: 398-421, 1997).

It is also known that the cell component formation rate is growth rate-dependent (Pramanik, J. and Keasling J.D., Biotechnol. Bioeng., 60:230-238, 1998). Further, the composition of cell components also depends on the growth rate, and by measuring it, it can be described with mathematical equations by using the specific growth rate (Bremer and Dennis, In Escherichia coli and Salmonella: Cellular and Molecular Biology/Second Edition (Neidhardt, F.C. Ed.), pp. 1553-1569., American Society for Microbiology Press, Washington, D.C., 1996).

With the cell component formation rate $V_{biomass}$ obtained as described above, the influence on intracellular metabolites can be taken into consideration for metabolites as precursors of the cell components. Specifically, by incorporating $V_{biomass}$ into $V_{output}$ in the differential equation of a metabolite as a precursor of the cell component (equation 1) to perform calculation, it becomes possible to describe the material balance of the metabolite as a precursor of the cell component with a growth rate factor equation.

If the formation rate of the cell component can be measured, it is also possible to represent values measured in a production process as a time function and use it directly. Moreover, if the function representing the formation rate is represented with a specific growth rate-dependent equation, the formation rate can be converted into a time function by substituting a time function of the specific growth rate into the specific growth rate-dependent equation. The specific growth rate may be the same as that used in (a) mentioned above, or a specific growth rate based on another index relating to growth of cells.

Moreover, it is preferable to represent the composition of cell components itself with a mathematical equation using the specific growth rate of cells or its equivalent index concerning the growth.

(d) Preparation of mathematical equations representing inflow rate of metabolites from outside of cells and outflow rate of metabolites out of cells

An inflow rate of a metabolite taken up from the outside of cells and/or an outflow rate of a metabolite excreted out of cells are incorporated into the differential equations, and the inflow rate and/or the outflow rate is represented as a growth rate factor.. As set forth above, the growth rate factor can be expressed as a function of time or as a function of the specific growth rate $\mu$.

In a production process of a substance, it is common to add an organic substance other than the substrate such as glucose as a medium component. Examples include tryptone, soybean hydrolysate, yeast extract and so forth. Substances such as amino acids derived from such medium components are also taken up into cells and affect the metabolic simulation. It is possible to describe an uptake rate of a metabolite taken up from the inside of cells $V_{uptake}$ with a function of the specific growth rate or time function based on measured values of concentrations of medium components remaining in the medium. As the metabolites excreted out of the cells from the inside of the cells during a production process, substances called by-products may also be excreted other than the objective product. By also incorporating these substances into the metabolic simulation, more accurate material balance can be described. The outflow rate to the outside of the cells Vexcretion can be represented with a mathematical equation using a growth rate factor. The growth rate factor can be a function of the specific growth rate or a time function from measured values of the metabolite concentration detected in the medium. By performing calculation with incorporating $V_{uptake}$ into $V_{input}$ and $V_{excretion}$ into $V_{input}$ in the differential equation of a metabolite (equation 1), the material balance depending on the growth rate of the cells or time can be described (Fig. 3).

If the inflow rate and/or the outflow rate can be measured, it is also possible to represent values measured in a production process as a time function and use it directly. For instance, Fig. 4 shows the concentration change curve, obtained by measurement of the extracellular acetic acid concentration, and the time equation of the excretion rate obtained therefrom. Moreover, if the function representing the inflow rate and/or the outflow rate is represented with a specific growth rate-dependent equation, the inflow rate and/or the outflow rate can be converted into a time function by substituting a time function of the specific growth rate into the specific growth rate-dependent equation. The specific growth rate may be the same as that used in (a) mentioned above, or a specific growth rate based on another index relating to growth of cells.

The metabolites taken up into the cells preferably are a substrate and/or an organic substance in the medium.

The substances excreted out of the cells preferably are an objective product and/or a by-product. The substances excreted out of the cells more preferably are amino acids, organic acids and/or carbon dioxide, further preferably amino acids or organic acids.

The cells used for the production process may be those of any type, so long as those used for substance production are chosen. Examples include, for example, various cultured cells, those of mold, yeast, various bacteria, and so forth. Preferred are those of a microorganism having an ability to produce a useful compound, for example, an amino acid, nucleic acid or organic acid. As a microorganism having an ability to produce an amino acid, nucleic acid or organic acid, E. *coli, Bacillus* bacteria, coryneform bacteria and so forth are preferably used. More preferred are those of a microorganism having an ability to produce an amino acid and/or an ability to produce an organic acid. The microorganism is preferably *Escherichia coli.*

By the simulation according to the method of the present invention, it becomes possible to predict dynamic behaviors of mRNA or protein concentrations for various enzymes in addition to intracellular metabolites. Therefore, the method of the present invention can serve as a useful tool in improvement of a production process of a useful substance, typically an amino acid or nucleic acid. For example, it becomes possible to verify effect of amplification or deletion of various enzymes in a computer (in silico experiments). Moreover, easy estimation of influence of change of parameters of various enzymes such as affinity to a substrate and affinity to an inhibitor on the whole metabolism and effect of amplification or deletion of a factor controlling expression of various enzyme genes also becomes possible. These results provide an important direction for improvement of a production process, and thus also have superior industrial usefulness.

The present invention further provides a program for executing the simulation method of the present invention and a storage means storing the program.

The program of the present invention is a program for making a computer execute the simulation method of the present invention and causes a computer to function as the following means (1) to (3):

(1) a means for storing a set of differential equations concerning intracellular metabolites and gene expression and satisfying the following (a) to (d);

(a) the differential equations include a specific growth rate of the cells, expressed as a differential equation wherein the specific growth rate is represented as a function of time;
(b) all or a part of the parameters of the differential equations are represented as a function of a growth rate factor.
(c) the differential equations include a formation rate for formation of a cell component from an intracellular metabolite, and the formation rate is represented as a function of a growth rate factor;
(d) the differential equations include an inflow rate of a metabolite taken up from the outside of the cells and/or an outflow rate of a metabolite excreted out of the cells from the inside of the cells, and the inflow rate and/or the outflow rate is represented as a growth rate factor;

(2) a means for storing values of parameters in the set of differential equations required for the simulation, and
(3) a means for calculating solutions of the set of differential equations based on the stored differential equations and values of the parameters.

A flowchart of a process executed by the program of the present invention is shown in Fig. 5. Moreover, a functional block diagram of a computer on which the program of the present invention is installed is shown in Fig. 6.

The means for storing a set of differential equations is constituted by a central processing portion 1, a storing portion 2 and an input portion 3. In a routine (S1) of storing a set of differential equations, the central processing portion 1 stores data of the set of differential equations inputted from the input portion 3 in the storing portion 2. The format of the data of the set of differential equations is not particularly limited, and it may be a usual format.

A means for storing values of parameters is constituted by the central processing portion 1, the storing portion 2 and the input portion 3. In a routine (S2) of storing values of parameters, the central processing portion 1 stores values of parameters inputted from the input portion 3 in the storing portion 2.

A means for computing solutions of the set of differential equations is constituted by the central processing portion 1, the storing portion 2 and an output portion 4. In a routine (S3) of computing the solutions of the set of differential equations, the central processing portion reads out the data of the differential equations and the values of parameters from the storing portion 2, computes the solutions from them and outputs the solutions to the output portion 4.

The central processing portion 1 is, for example, a processor. The storing portion 2 is, for example, a storage device using a recording medium. The input portion 3 is, for example, an input device such as keyboard and other devices or a data receiver for data from another device. The output portion 4 is, for example, an output device such as display, or a data transmission device for transmission to other devices.

The program for causing a computer to function as the aforementioned means can be created according to a usual programming method.

Further, the program of the present invention can also be stored in a computer-readable recording medium. The "recording medium" referred to herein include any "transportable physical media" such as floppy disk (registered trademark), magneto-optical disk, ROM, EPROM, EEPROM, CD-ROM, MO and DVD, any "physical media for fixation" such as ROM, RAM and HD built in various computer systems, and "communication media" storing the program over a short period of time such as communication cables and carrier waves in the case of transmitting the program through a network, of which typical examples are LAN, WAN and Internet.

Further, the "program" is a data processing method described with any language or description mode, and the format such as source code and binary code is not limited. In addition, a "program" is not necessarily' restricted to those configured as a single program, and includes those configured as a distributed system as two or more modules and libraries and those achieving the function through cooperation with another program, of which typical example is an operation system (OS). As specific configuration for reading from the recording medium in the devices shown in the embodiment, routines for reading, routines for installation after reading and so forth, known configurations and routines can be used.

The present invention provides accurate metabolic simulation results for a substance-production process that is accompanied by growth of cells. A simulation of the invention thus enables in silico experiments, which lend practical direction to improving such a production process, typically for obtaining an amino acid or nucleic acid. Illustrative of the improvement realizable in this regard is production optimization in batch culture or fed-batch culture often used as actual industrial process.

<Example 1>

Hereafter, the present invention will be further explained with reference to examples.

<1> System parameters

Simulation of expression of the proteins of the enzymes and transcription factors from the genes and conversion of substances by the enzymatic reactions mentioned in the central metabolic map of *E. coli* shown in Fig. 7 was performed. The system parameters used for the simulation and the metabolites of which quantities were included as constants are shown in Table 1. In order to use experimental values of turbidity OD (optical density) as an index of growth, the following basic data were obtained. For dry cell weight per OD, DCWperOD, the results obtained in weight measurement of dry cells obtained from 300 ml of culture broth of MG1655 strain by centrifugal separation. As for cell density per OD, celldens, cell density of the culture broth of MG1655 strain subjected to two steps of dilution was measured 5 times, this procedure was independently repeated 5 times (measurement was performed for 50 plates in total), and the average of the results was used. Cell volume, cellvol, and cell weight, cellweight, per cell were calculated by considering that cellvol of per 1 g of DCW was 0.0025 l/g (Rohwer et al., J. Biol. Chem., 275, 34909-34921, 2000). The translation rate constant was calculated from a value mentioned in literature, 11.03 (min)$^{-1}$, at $\mu$ of 0.01 (min)$^{-1}$ (Lee and Baily, Biotech. Bioeng., 26, 66-72, 1984) and a ribosome concentration. As the rate constant of proteolysis, a value mentioned in literature was used either (Miller, C.G., In Escherichia coli and Salmonella: Cellular and Molecular Biology/Second Edition (Neidhardt, F.C. Ed.), pp.680-691, American Society for Microbiology Press, Washington, D.C., 1996).

Table 1: System parameters

| The system parameters and values of the quantities of metabolites included as constants are shown. The values for which titles of literature are mentioned are literature values, and "Experimental" indicates that the values were obtained by experiments. | | | | |
|---|---|---|---|---|
| System parameter | Name | Value | Unit | Reference |
| DCWperOD | dry cell weight per OD | 3.91E-01 | g/L | Experimental |
| celldens | cell density | 3.99E+12 | cells/L | Experimental |
| reacvol | culture volume | 3.00E-01 | L | Experimental |
| initOD | initilal OD | 2.07E+00 | | Experimental |
| cellvol | volume of single cell | 2.00E-16 | L | Calculated from gDCW/cellvol (0.0025 l/g) |
| cellweight | weight of single cell | 8.00E-14 | g | Calculated from gDCW/cellvol (0.0025 l/g) |
| ktrans | rate constant for translation | 1.21E+05 | (Mmin)$^{-1}$ | Biotech. Bioeng. 26, 66-72, 1984 |

(continued)

| The system parameters and values of the quantities of metabolites included as constants are shown. The values for which titles of literature are mentioned are literature values, and "Experimental" indicates that the values were obtained by experiments. | | | | |
|---|---|---|---|---|
| System parameter | Name | Value | Unit | Reference |
| kdeg | rate constant for protein degradation | 1.67E-04 | min$^{-1}$ | Escherichia coli and Salmonella 44, 680-691, 1996 |
| ATP | adenosine-5-triphosphate | 4.27E-03 | M | Biotech Bioeng. 79, 53-73, 2002 |
| ADP | adenosine-5-diphosphate | 5.95E-04 | M | Biotech. Bioeng. 79, 53-73, 2002 |
| AMP | adenosine-5-monophosphate | 9.55E-04 | M | Biotech. Bioeng. 79, 53-73, 2002 |
| NAD | nicotinamide adenine dinucleotide | 1.47E-03 | M | Biotech. Bioeng. 79, 53-73, 2002 |
| NADH | nicotinamide adenine dinucleotide reduced | 1.00E-04 | M | Biotech. Bioeng. 79, 53-73, 2002 |
| NADP | dihydronicotinamide adenine dinucleotide phosphate | 1.95E-04 | M | Biotech. Bioeng. 79, 53-73, 2002 |
| NADPH | dihydronicotinamide adenine dinucleotide phosphate reduced | 6.20E-05 | M | Biotech. Bioeng. 79, 53-73, 2002 |
| CoA | CoA | 1.23E-04 | M | Metab. Eng. 4, 182-192, 2002 |
| Pi | inorganic phosphate | 1.00E-02 | M | Escherichia coli and Salmonella 87, 1357-1381, 1996 |
| ASP | aspartate | 1.34E-03 | M | Biochem. J. 356, 433-444, 2001 |
| CO2 | carbon dioxide | 1.35E-03 | M | Experimental |

<2> Modeling of enzymatic reaction

The abbreviations and initial values of the metabolites used in this example are shown in Table 2. As for those obtained from literature, titles of literature are shown. In the simulation, supposing that the volumes of all cells increased in the process of growth, the total cell volume (cellvoltot) was used as a variable. The initial value of the total cell volume (cellvoltot) was calculated from the initially measured value of OD (initOD) in accordance with the following equation.

$$\texttt{cellvoltot = cellvol x celldens x reacvol x initOD}$$

Modeling of the enzymatic reactions was performed based on the Michaelis-Menten type equation described by Segel (Segel, Enzyme Kinetics: Behavior and Analysis of Rapid Equilibrium and Steady-State Enzyme Systems, John Wiley & Sons, New York, 1975). Names and initial values of quantities of enzymes, transcription factors and mRNA are shown in Table 3. Types, values of parameters, substrates, products and effectors of the enzymatic reactions are shown in Table 4. The enzymatic reaction formulas are shown in Table 4.

Table 2: Abbreviations and initial values of metabolites The values for which no literature name is mentioned are estimated values.

| Variable | Name | Initial value | Unit | Reference |
|---|---|---|---|---|
| Cellvoltot | total cell volume | 4.96E-04 | L | |
| Glext | external glucose | 2.20E-01 | M | Experimental |
| G6P | glucose-6-phosphate | 3.48E-03 | M | Biotech. Bioeng. 79, 53-73, 2002 |
| F6P | fructose-6-phosphate | 6.00E-04 | M | Biotech. Bioeng. 79, 53-73, 2002 |
| FDP | fructose-16-diphosphate | 2.72E-04 | M | Biotech. Bioeng. 79, 53-73, 2002 |
| GA3P | glyceraldehyde 3-phosphate | 2.18E-04 | M | Biotech. Bioeng. 79, 53-73, 2002 |
| DHAP | dihydroxyacetone phosphate | 1.67E-04 | M | Biotech. Bioeng. 79, 53-73, 2002 |
| 13DPG | 13-bis-phosphoglycerate | 8.00E-06 | M | Biotech. Bioeng. 79, 53-73, 2002 |
| 3PG | 3-phosphoglycerate | 2.50E-03 | M | Nature 305, 286-290, 1983 |
| 2PG | 2-phosphoglycerate | 3.99E-04 | M | Biotech. Bioeng. 79, 53-73, 2002 |
| PEP | phosphoenolpyruvate | 2.67E-03 | M | Biotech. Bioeng. 79, 53-73, 2002 |
| PYR | pyruvate | 2.67E-03 | M | Biotech. Bioeng. 79, 53-73,2002 |
| 6PGC | 6-phosphogluconate | 8.08E-04 | M | Biotech. Bioeng. 79, 53-73, 2002 |
| RL5P | ribulose-5-phsophate | 1.11E-04 | M | Biotech. Bioeng. 79, 53-73, 2002 |
| R5P | ribose-5-phosphate | 3.98E-04 | M | Biotech. Bioeng. 79, 53-73, 2002 |
| X5P | xylulose-5-phosphate | 1.38E-04 | M | Biotech. Bioeng. 79, 53-73, 2002 |
| B4P | erythrose-4-phosphate | 9.80E-05 | M | Biotech. Bioeng. 79, 53-73, 2002 |
| S7P | sedoheptulose-7-phosphate | 2.76E-04 | M | Biotech. Bioeng. 79, 53-73, 2002 |
| ACCoA | acetylCoA | 3.00E-04 | M | Anal. Biochem. 295, 129-137, 2001 |
| OAA | oxaloacetate | 6.80E-04 | M | Biomol. Eng. 19, 5-15, 2002 |
| CIT | citrate | 1.50E-04 | M | Biomol. Eng. 19, 5-15, 2002 |
| ICIT | isocitrate | 1.70E-04 | M | Biomol. Eng. 19, 5-15, 2002 |

(continued)

| Variable | Name | Initial value | Unit | Reference |
|---|---|---|---|---|
| AKG | 2-ketoglutarate | 1.80E-04 | M | Biomol. Eng. 19, 5-15, 2002 |
| SUCCoA | succinylCoA | 1.00E-04 | M | |
| SUCC | succinate | 1.90E-04 | M | Biomol. Eng. 19, 5-15, 2002 |
| FUM | fumarate | 1.00E-04 | M | |
| MAL | malate | 6.00E-05 | M | Biomol. Eng. 19, 5-15, 2002 |
| GLX | glyoxylate | 1.00E-04 | M | |
| cAMP | cyclic AMP | 8.00E-06 | M | Mol. Microbiol. 10, 341-350, 1993 |
| cAMPxt | external cyclic AMP | 8.00E-06 | M | Mol. Microbiol. 10, 341-350, 1993 |
| FIP | fructose 1-phosphate | 1.00E-04 | M | |
| CO2xt | external CO2 | 0.00E+00 | M | |

Table 3: Initial concentrations of enzymes and transcription factors
The values changed during the simulation are specified in the column of "fold-change".

| Variable | Name | Initial value | fold-change | Unit | Reference |
|---|---|---|---|---|---|
| crpmRNA | mRNA of crp gene | 0.00E+00 | | M | |
| CRP | cAMP receptor protein | 1.15E-05 | | M | Mol. Microbiol. 10, 341-350, 1993 |
| mlcmRNA | mRNA of mlc gene | 0.00E+00 | | M | |
| Mlc | Mlc protein | 7.10E-08 | | M | EMBO J. 20, 5344-5352, 2000 |
| cyaAmRNA | mRNA of cyaA gene | 0.00E+00 | | M | |
| CYA | adenylate cyclase | 8.50E-08 | | M | J. Biol. Chem. 258, 3750-3758, 1983 |
| cpdAmRNA | mRNA of cpdA gene | 0.00E+00 | | M | |
| CPD | cAMP phosphodiesterase | 8.08E-06 | | M | J. Bacteriol. 116, 857-866, 1973 |
| ptsHImRNA | mRNA of ptsHI gene | 0.00E+00 | | M | |
| EItot | enzyme I | 1.12E-05 | | M | Can. J. Biochem. Cell Biol. 61, 29-37, 1983 |
| EI-P | phosphorylated EI | 1.06E-05 | | M | |
| HPrtot | enzyme HPr | 1.23E-04 | | M | Can. J. Biochem. Cell Biol. 61, 29-37, 1983 |
| HPr-P | phosphorylated HPr | 1.17E-04 | | M | |
| crrmRNA | mRNA of crr gene | 0.00E+00 | | M | |
| IIAGlctot | enzyme IIAGlc | 7.69E-05 | | M | J. Bacteriol. 148 257-264, 1981 |
| IIAGlc-P | phosphorylated IIAGlc | 7.31E-05 | | M | |
| ptsGmRNA | mRNA of ptsG gene | 0.00E+00 | | M | |
| IICBGlctot | enzyme IICBGlc | 7.21E-06 | 1.5 | M | Proc. Natl. Acad. Sci. USA. 84, 930-934, 1987 |
| IICBGlcP | phosphorylated IICBGlcP | 6.85E-06 | 1.5 | M | |
| pgimRNA | mRNA of pgi gene | 0.00E+00 | | M | |
| PGI | phosphoglucose isomerase | 1.85E-05 | | M | Arch. Microbiol. 127, 289-298, 1980 |
| pfkAmRNA | mRNA of pfkA gene | 0.00E+00 | | M | |
| PFKA | phosphofructokinase I | 1.42E-06 | 3.5 | M | Methods Enzymol. 90, 60-70, 1982 |
| fbamRNA | mRNA of fba gene | 0.00E+00 | | M | |
| FBA | fructose-16-bisphosphatate aldolase II | 3.09E-05 | | M | Biochem. J. 169, 633-641, 1978 |
| tpiAmRNA | mRNA of tpiA gene | 0.00E+00 | | M | |
| TPIA | triosphosphate isomerase | 5.68E-05 | | M | J. Biol. Chem. 270, 29096-29104, 1995 |
| gapAmRNA | mRNA of gapA gene | 0.00E+00 | | M | |
| GAPA | glyceraldehyde-3-phosphate dehydrogenase-A | 3.56E-05 | | M | Biochem. J. 179, 99-107, 1979 |
| pgkmRNA | mRNA of pgk gene | 0.00E+00 | | M | |
| PGK | phosphoglycerate kinase | 4.92E-05 | | M | J. Biol. Chem. 246, 4319-4325, 1971 |

Table 3 (continued)

| Variable | Name | Initial value | fold-change | Unit | Reference |
|---|---|---|---|---|---|
| pgmARNA | mRNA of pgmA gene | 0.00E+00 | | M | |
| dPGM | phosphoglycerate mutase d | 8.80E-05 | | M | FEBS Lett. 455, 344-348, 1999 |
| yibOmRNA | mRNA of yibO gene | 0.00E+00 | | M | |
| iPGM | phosphoglycerate mutase i | 2.22E-05 | | M | FEBS Lett. 455, 344-348, 1999 |
| enomRNA | mRNA of eno gene | 0.00E+00 | | M | |
| ENO | enolase | 7.65E-05 | | M | J. Biol. Chem. 246, 6797-6802, 1971 |
| pykFmRNA | mRNA of pykF gene | 0.00E+00 | | M | |
| PYKF | pyruvate kinase I | 2.75E-06 | | M | Methods Enzymol. 90, 170-179, 1982 |
| zwfmRNA | mRNA of zwf gene | 0.00E+00 | | M | |
| G6PD | glucose-6-phosphate dehydrogenase | 1.04E-05 | | M | J. Bacteriol. 110, 155-160, 1972 |
| gndmRNA | mRNA of gnd gene | 0.00E+00 | | M | |
| 6PGD | 6-phosphogluconate dehydrogenase | 1.85E-05 | | M | J. Bacteriol. 138, 171-175, 1979 |
| rpiAmRNA | mRNA of rpiA gene | 0.00E+00 | | M | |
| RPIA | ribose-5-phosphate isomerase I | 3.66E-06 | | M | J. Bacteriol. 175, 5628-5635, 1993 |
| rpemRNA | mRNA of rpe gene | 0.00E+00 | | M | |
| RPE | ribulose-5-phsophate 3-epimerase | 6.21E-06 | | M | |
| talBmRNA | mRNA of talB gene | 0.00E+00 | | M | |
| TALB | transaldolase B | 5.94E-06 | | M | J. Bacteriol. 177, 5930-5936, 1995 |
| tktAmRNA | mRNA of tktA gene | 0.00E+00 | | M | |
| TKTA | transketolase A | 3.46E-06 | | M | Eur. J. Biochem. 230, 525-532, 1995 |
| pdhRaceEFmRNA | mRNA of pdhRaceEF gene | 0.00E+00 | | M | |
| PdhR | pyruvate dehydrogenase complex repressor | 6.66E-08 | | M | Mol. Microbiol. 15, 519-529, 1995 |
| PDH | pyruvate dehydrogenase | 3.32E-07 | | M | Methods Enzymol. 89, 391-399, 1982 |
| gltAmRNA | mRNA of gltA gene | 0.00E+00 | | M | |
| CS | citrate synthase | 3.68E-06 | 3 | M | Biochemistry 8, 4497-4503, 1969 |
| acnAmRNA | mRNA of acnA gene | 0.00E+00 | | M | |
| ACNA | aconitase A | 4.19E-06 | 1.7 | M | J. Gen. Microbiol. 137, 2505-2515, 1991 |
| acnBmRNA | mRNA of acnB gene | 0.00E+00 | | M | |
| ACNB | aconitase B | 1.13E-05 | 1.7 | M | Microbiology 142, 389-400, 1996 |
| icdAmRNA | mRNA of icdA gene | 0.00E+00 | | M | |
| ICDA | isocitrate dehydrogenase | 1.19E-04 | 0.5 | M | J. Biol. Chem. 254, 7915-7920, 1979 |

Table 3 (continued)

| Variable | Name | Initial value | fold-change | Unit | Reference |
|---|---|---|---|---|---|
| sucABCDmRNA | mRNA of suABCD gene | 0.00E+00 | | M | |
| KGDH | α-ketoglutarate dehydrogenase | 7.04E-06 | 0.8 | M | MethodsEnzymol. 13, 55-61, 1969 |
| SCS | succinyl-CoA synthetase | 3.03E-05 | 1.5 | M | J. Biol. Chem. 242, 4287-4298, 1967 |
| sdhCDABmRNA | mRNA of sdhCDAB gene | 0.00E+00 | | M | |
| SDH | succinate dehydrogenase | 5.08E-05 | | M | J. Biol. Chem. 264, 2672-2677, 1989 |
| frdABCDmRNA | mRNA of frdABCD gene | 0.00E+00 | | M | |
| FRD | fumarate reductase aerobic | 5.40E-06 | | M | Methods Enzymol. 126, 377-386, 1986 |
| fumAmRNA | mRNA of fumA gene | 0.00E+00 | | M | |
| FUMA | fumarase A Class I, aerobic | 2.10E-06 | | M | Biochemistry 31, 10331-10337, 1992 |
| mdhmRNA | mRNA of mdh gene | 0.00E+00 | | M | |
| MDH | malate dehydrogenase | 1.44E-05 | 5 | M | J. Bacteriol. 163, 1074-1079, 1985 |
| fbpmRNA | mRNA of fbp gene | 0.00E+00 | | M | |
| FBP | fructose-16-bisphosphatase | 2.55E-07 | | M | Arch. Biochem. Biophys. 225, 944-949, 1983 |
| ppsAmRNA | mRNA of ppsA gene | 0.00E+00 | | M | |
| PPS | phsophoenolpyruvate synthase | 3.86E-06 | 0.02 | M | J. Biol. Chem. 245, 5309-5318, 1970 |
| scfAmRNA | mRNA of scfA gene | 0.00E+00 | | M | |
| NADME | NAD-dependent malic enzyme | 2.75E-07 | 0.1 | M | J. Biochem. 73, 169-180, 1973 |
| b2463mRNA | mRNA of b2463 gene | 0.00E+00 | | M | |
| NADPME | NADP-dependent malic enzyme | 1.87E-07 | 0.1 | M | J. Biochem. 85, 1355-1365, 1979 |
| ppcmRNA | mRNA of ppc gene | 0.00E+00 | | M | |
| PPC | PEP carboxylase | 1.89E-06 | 10 | M | J. Biol. Chem. 247, 5785-5792, 1972 |
| pckAmRNA | mRNA of pckA gene | 0.00E+00 | | M | |
| PCK | PEP carboxykinase ATP | 1.08E-06 | | M | J. Biol. Chem. 255, 1399-1405, 1980 |
| iclRmRNA | mRNA of iclR gene | 0.00E+00 | | M | |
| IclR | acetate operon repressor | 8.30E-08 | | M | |
| aceBAKmRNA | mRNA of aceBAK gene | 0.00E+00 | | M | |
| ICL | isocitrate Lyase | 1.20E-05 | 0.1 | M | Biochem. J. 250, 25-31, 1988 |
| MSA | malate synthase A | 3.61E-06 | 0.1 | M | J. Bacteriol. 175, 4572-4575, 1993 |
| ICDKP | isocitrate dehydrogenase kinase/phosphatase | 3.61E-08 | 0.1 | M | J. Bacteriol. 175, 4572-4575, 1993 |
| FRUK | fructose 1-phosphate kinase | 7.41E-06 | | M | J. Bacteriol. 172, 5459-5469, 1990 |

EP 1 752 899 A1

Table 4: Types, parameters, substrates, products and effectors of enzymatic reactions
The values changed during the simulation are specified in the column of "fold-change".

| Enzyme | Type | Parameter | Value | fold-change | Unit | Substrate | Product | Effector | Reference |
|---|---|---|---|---|---|---|---|---|---|
| Glucose PTS | fbmm | k1f1 | 1.08E+05 | | min$^{-1}$ | PEP | PYR | | J. Biol. Chem. 275, 34909-34921, 2000 |
| r1a | | k1r1 | 4.80E+05 | | min$^{-1}$ | EI | EIP | | J. Biol. Chem. 275, 34909-34921, 2000 |
| | | m1s1 | 3.00E-04 | | M | | | | J. Biol. Chem. 275, 34909-34921, 2000 |
| | | m1p1 | 2.00E-03 | | M | | | | J. Biol. Chem. 275, 34909-34921, 2000 |
| Glucose PTS | ma2 | k1a | 1.20E+10 | | min$^{-1}$ | EIP | EI | | J. Biol. Chem. 275, 34909-34921, 2000 |
| r1b | ma2 | k1b | 4.80E+08 | | min$^{-1}$ | HPr | HPrP | | J. Biol. Chem. 275, 34909-34921, 2000 |
| Glucose PTS | ma2 | k1c | 3.66E+09 | | min$^{-1}$ | HPrP | HPr | | J. Biol. Chem. 275, 34909-34921, 2000 |
| r1c | | k1d | 2.92E+09 | | min$^{-1}$ | IIAGlc | IIAGlcP | | J. Biol. Chem. 275, 34909-34921, 2000 |
| Glucose PTS | ma2 | k1e | 6.60E+08 | | min$^{-1}$ | IIAGlcP | IIAGlc | | J. Biol. Chem. 275, 34909-34921, 2000 |
| r1d | | k1g | 2.40E+08 | | min$^{-1}$ | IICBGlc | IICBGlcP | | J. Biol. Chem. 275, 34909-34921, 2000 |
| Glucose PTS | smm | k1f2 | 4.80E+03 | | min$^{-1}$ | IICBGlcP | G6P | | J. Biol. Chem. 275, 34909-34921, 2000 |
| r1e | | m1s2 | 2.00E-05 | | M | GLC | IICBGlc | | J. Biol. Chem. 275, 34909-34921, 2000 |
| PGI | revuumm | k2f | 9.54E+04 | | min$^{-1}$ | G6P | F6P | | Experimental |
| r2 | | k2r | 1.92E+04 | | min$^{-1}$ | | | | Experimental |
| | | m2s | 4.70E-03 | | | | | | Experimental |
| | | m2p | 5.14E-04 | | | | | | Experimental |
| PFKA | csmm | k3f | 1.00E+04 | | min$^{-1}$ | F6P | FDP | | J. Biol. Chem. 269, 18475-18479, 1994 |
| r3 | | m3s1 | 1.40E-04 | | M | ATP | ADP | | FEBS. Lett. 290, 173-176, 1991 |
| | | m3s2 | 1.50E-04 | | M | | | | FEBS. Lett. 290, 173-176, 1991 |
| | | n3 | 1.00E+00 | | | | | | FEBS. Lett. 290, 173-176, 1991 |
| FBA | ordub | k4f | 1.82E+03 | 10 | min$^{-1}$ | FDP | DHAP | | FEBS. Lett. 318, 11-16, 1993 |
| r4 | | k4r | 2.20E+03 | | min$^{-1}$ | | GA3P | | Biochemistry 32, 4685-4692, 1993 |
| | | k4eq | 1.00E-04 | 10 | M | | | | Biochemistry 32, 4685-4692, 1993 |
| | | m4s | 1.33E-04 | | M | | | | Biochemistry 32, 4685-4692, 1993 |
| | | m4p1 | 8.80E-05 | | M | | | | Biochemistry 32, 4685-4692, 1993 |
| | | m4p2 | 8.80E-05 | | M | | | | Biochemistry 32, 4685-4692, 1993 |
| | | k4i | 6.00E-04 | | M | | | | Biochemistry 32, 4685-4692, 1993 |
| TPIA | revuumm | k5f | 3.86E+03 | 2000 | min$^{-1}$ | DHAP | GA3P | | Experimental |
| r5 | | k5r | 1.56E+05 | 0.0005 | min$^{-1}$ | | | | Experimental |
| | | m5s | 4.91E-04 | | M | | | | Experimental |
| | | m5p | 2.89E-02 | | M | | | | Experimental |
| | | n5 | 1.90E+00 | | M | | | | Experimental |

EP 1 752 899 A1

Table 4 (continued)

| Enzyme | Type | Parameter | Value | fold-change | Unit | Substrate | Product | Effector | Reference |
|---|---|---|---|---|---|---|---|---|---|
| GAPA | revtbmm | k6f | 6.34E+04 | 2 | min$^{-1}$ | GA3P | 13DPG | | Biochemistry 28 2586-2592, 1989 |
| r6 | | k6r | 5.40E+04 | | min$^{-1}$ | NAD | NADH | | Biochemistry 28 2586-2592, 1989 |
| | | m6s1 | 1.50E-03 | | M | PI | | | Biochemistry 28 2586-2592, 1989 |
| | | m6s2 | 4.20E-05 | | M | | | | Biochemistry 28 2586-2592, 1989 |
| | | m6s3 | 2.20E-02 | | M | | | | Biochemistry 28 2586-2592, 1989 |
| | | m6p1 | 1.50E-05 | | M | | | | Biochemistry 28 2586-2592, 1989 |
| | | m6p2 | 1.20E-05 | | M | | | | Eur. J. Biochem. 198, 429-435, 1991 |
| PGK | revbbmm | k7f | 3.02E+04 | | min$^{-1}$ | 13DPG | 3PG | | Experimental |
| r7 | | k7r | 1.18E+04 | | min$^{-1}$ | ADP | ATP | | Experimental |
| | | m7s1 | 4.44E-06 | | M | | | | Experimental |
| | | m7s2 | 4.06E-05 | | M | | | | Experimental |
| | | m7p1 | 3.65E-04 | | M | | | | Experimental |
| | | m7p2 | 1.77E-04 | | M | | | | Experimental |
| dPGM | revuumm | k8f | 1.98E+04 | | min$^{-1}$ | 3PG | 2PG | | FEBS Lett. 455, 344-348, 1999 |
| fr8 | | k8r | 1.32E+04 | | min$^{-1}$ | | | | FEBS Lett. 455, 344-348, 1999 |
| | | m8s | 2.00E-04 | | M | | | | FEBS Lett. 455, 344-348, 1999 |
| | | m8p | 1.90E-04 | | M | | | | FEBS Lett. 455, 344-348, 1999 |
| iPGM | revuumm | k9f | 1.32E+03 | | min$^{-1}$ | 3PG | 2PG | | FEBS Lett. 455, 344-348, 1999 |
| r9 | | k9r | 6.00E+02 | | min$^{-1}$ | | | | FEBS Lett. 455, 344-348, 1999 |
| | | m9s | 2.10E-04 | | M | | | | FEBS Lett. 455, 344-348, 1999 |
| | | m9p | 9.70E-05 | | M | | | | FEBS Lett. 455, 344-348, 1999 |
| ENO | revuumm | k10f | 6.24E+03 | | min$^{-1}$ | 2PG | PEP | | Experimental |
| r10 | | k10r | 2.21E+03 | | min$^{-1}$ | | | | Experimental |
| | | m10s | 7.15E-06 | | M | | | | Experimental |
| | | m10p | 7.15E-05 | | M | | | | Experimental |
| PYKF | csmm | k11f | 9.60E+03 | | min$^{-1}$ | PEP | PYR | | J. Biol. Chem. 275 18145-18152, 2000 |
| r11 | | m11s1 | 8.00E-05 | | M | ADP | ATP | | J. Biol. Chem. 275 18145-18152, 2000 |
| | | m11s2 | 3.00E-04 | | M | | | | J. Biol. Chem. 275 18145-18152, 2000 |
| | | n11 | 1.00E+00 | | | | | | J. Biol. Chem. 275 18145-18152, 2000 |
| G6PD | sbmm | k12f | 1.18E+04 | | min$^{-1}$ | NADP | NADPH | | Experimental |
| r12 | | m12s1 | 3.00E-04 | | M | G6P | 6PGC | | Experimental |
| | | m12s2 | 3.74E-03 | | M | | | | Experimental |
| 6PGD | sbmm | k13f | 3.25E+03 | | min$^{-1}$ | NADP | NADPH | | Experimental |
| r13 | | m13s1 | 1.67E-04 | | M | 6PGC | RL5P | | Experimental |
| | | m13s2 | 1.31E-04 | | M | | CO2 | | Experimental |
| RPIA | revuumm | k14f | 2.38E+05 | | min$^{-1}$ | RL5P | R5P | | Experimental |
| r14 | | k14r | 4.89E+02 | 20 | min$^{-1}$ | | | | Experimental |
| | | m14s | 1.17E-02 | | M | | | | Experimental |
| | | m14p | 8.72E-04 | | M | | | | Experimental |

Table 4 (continued)

| Enzyme | Type | Parameter | Value | fold-change | Unit | Substrate | Product | Effector | Reference |
|---|---|---|---|---|---|---|---|---|---|
| RPE<br>rl5 | revuumm | kl5f | 1.32E+04 | | min⁻¹ | RL5P | X5P | | Experimental |
| | | kl5r | 2.10E+03 | 5 | min⁻¹ | | | | |
| | | ml5s | 5.15E-03 | | M | | | | Experimental |
| | | ml5p | 8.90E-04 | | M | | | | |
| TALB<br>rl6 | revbbmm | kl6f | 2.10E+02 | 100 | min⁻¹ | S7P | E4P | | J. Bacteriol. 177, 5930-5936, 1995 |
| | | kl6r | 5.60E+03 | | min⁻¹ | GA3P | F6P | | J. Bacteriol. 177, 5930-5936, 1995 |
| | | ml6s1 | 2.85E-04 | | M | | | | J. Bacteriol. 177, 5930-5936, 1995 |
| | | ml6s2 | 3.80E-05 | | M | | | | J. Bacteriol. 177, 5930-5936, 1995 |
| | | ml6p1 | 9.00E-05 | | M | | | | J. Bacteriol. 177, 5930-5936, 1995 |
| | | ml6p2 | 1.20E-03 | | M | | | | J. Bacteriol. 177, 5930-5936, 1995 |
| TKTI<br>rl7 | revbbmm | kl7f | 8.67E+02 | 100 | min⁻¹ | S7P | R5P | | Eur. J. Biochem. 230, 525-532, 1995 |
| | | kl7r | 7.28E+03 | | min⁻¹ | GA3P | X5P | | Eur. J. Biochem. 230, 525-532, 1995 |
| | | ml7s1 | 4.00E-03 | | M | | | | Eur. J. Biochem. 230, 525-532, 1995 |
| | | ml7s2 | 2.10E-03 | | M | | | | Eur. J. Biochem. 230, 525-532, 1995 |
| | | ml7p1 | 1.40E-03 | | M | | | | Eur. J. Biochem. 230, 525-532, 1995 |
| | | ml7p2 | 1.60E-04 | | M | | | | Eur. J. Biochem. 230, 525-532, 1995 |
| TKTII<br>rl7b | revbbmm | kl7f2 | 1.59E+04 | | min⁻¹ | X5P | F6P | | Eur. J. Biochem. 230, 525-532, 1995 |
| | | kl7r2 | 8.95E+03 | 5 | min⁻¹ | E4P | GA3P | | Eur. J. Biochem. 230, 525-532, 1995 |
| | | ml7s3 | 1.60E-04 | | M | | | | Eur. J. Biochem. 230, 525-532, 1995 |
| | | ml7s3 | 9.00E-05 | | M | | | | Eur. J. Biochem. 230, 525-532, 1995 |
| | | ml7p3 | 1.10E-03 | | M | | | | Eur. J. Biochem. 230, 525-532, 1995 |
| | | ml7p4 | 2.10E-03 | | M | | | | Eur. J. Biochem. 230, 525-532, 1995 |
| PDH<br>rl8 | csmm | kl8f | 2.45E+04 | | min⁻¹ | PYR | ACCoA | | Biochemistry 19, 4208-4213, 1980 |
| | | ml8s1 | 1.76E-04 | | M | NAD | NADH | | Biochemistry 19, 4208-4213, 1980 |
| | | ml8s2 | 4.10E-04 | | M | CoA | CO2 | | Biochemistry 19, 4208-4213, 1980 |
| | | n18 | 1.00E+00 | | | | | | Biochemistry 19, 4208-4213, 1980 |
| CS<br>r19 | irrord | kl9f | 4.86E+03 | | min⁻¹ | OAA | CIT | | J. Biol. Chem. 263, 2163-2169, 1988 |
| | | ml9s1 | 2.60E-05 | | M | ACCoA | CoA | | J. Biol. Chem. 263, 2163-2169, 1988 |
| | | ml9s2 | 1.20E-04 | | M | | | | J. Biol. Chem. 263, 2163-2169, 1988 |
| | | ki19s | 3.30E-05 | | M | | | | J. Biol. Chem. 263, 2163-2169, 1988 |
| ACNA<br>r20 | revuumm | k20f | 5.98E+02 | | min⁻¹ | CIT | ICIT | | Biochem. J. 344, 739-746, 1999 |
| | | k20r | 1.43E+03 | | min⁻¹ | | | | Biochem. J. 344, 739-746, 1999 |
| | | m20s | 1.16E-03 | | M | | | | Biochem. J. 344, 739-746, 1999 |
| | | m20p | 1.77E-03 | | M | | | | Biochem. J. 344, 739-746, 1999 |

Table 4 (continued)

| Enzyme | Type | Parameter | Value | fold-change | Unit | Substrate | Product | Effector | Reference |
|---|---|---|---|---|---|---|---|---|---|
| ACNB r21 | revuumm | k21f | 2.23E+03 | | min⁻¹ | CIT | ICIT | | Biochem. J. 344, 739-746, 1999 |
| | | k21r | 5.40E+03 | | min⁻¹ | | | | Biochem. J. 344, 739-746, 1999 |
| | | m21s | 1.10E-02 | | M | | | | Biochem. J. 344, 739-746, 1999 |
| | | m21p | 1.97E-02 | | M | | | | Biochem. J. 344, 739-746, 1999 |
| ICDA r22 | icdbt | k22f | 4.83E+03 | | min⁻¹ | ICIT NADP | AKG NADPH CO2 | | Biochemistry 32, 9302-9309, 1993 |
| | | m22s1 | 1.10E-05 | | M | | | | Biochemistry 32, 9302-9309, 1993 |
| | | m22s2 | 1.70E-05 | | M | | | | Biochemistry 32, 9302-9309, 1993 |
| | | ki22s | 4.00E-06 | | M | | | | Biochemistry 32, 9302-9309, 1993 |
| | | k22r | 8.94E+02 | | min⁻¹ | | | | Biochemistry 32, 9302-9309, 1993 |
| | | m22p1 | 5.70E-04 | | M | | | | Biochemistry 32, 9302-9309, 1993 |
| | | m22p2 | 7.00E-06 | | M | | | | Biochemistry 32, 9302-9309, 1993 |
| | | m22p3 | 3.13E-03 | | M | | | | Biochemistry 32, 9302-9309, 1993 |
| | | c23a | 5.22E-07 | | M² | | | | Biochemistry 32, 9302-9309, 1993 |
| | | c22b | 4.18E-06 | | M² | | | | Biochemistry 32, 9302-9309, 1993 |
| | | c22c | 5.20E-08 | | M² | | | | Biochemistry 32, 9302-9309, 1993 |
| | | c22d | 1.50E-10 | | M³ | | | | Biochemistry 32, 9302-9309, 1993 |
| KGDH r23 | kgdhccs | k23f | 8.70E+03 | | min⁻¹ | AKG NAD CoA | SUCCoA NADH CO2 | | Biochemistry 23, 3136-3143, 1984 |
| | | alp23 | 4.97E-01 | | | | | | Biochemistry 23, 3136-3143, 1984 |
| | | m23s | 1.86E-05 | | M | | | | Biochemistry 23, 3136-3143, 1984 |
| | | kk23 | 3.90E-01 | | M | | | | Biochemistry 23, 3136-3143, 1984 |
| SCS r24 | revttmm | k24f | 2.78E+03 | | min⁻¹ | SUCCoA ADP PI | SUC ATP CoA | | Can. J. Biochem. 51, 44-55, 1973 |
| | | k24r | 3.99E+03 | | min⁻¹ | | | | J. Biol. Chem. 245, 2758-2762, 1970 |
| | | m24s1 | 7.70E-06 | | M | | | | Can. J. Biochem. 51, 44-55, 1973 |
| | | m24s2 | 1.20E-05 | | M | | | | Can. J. Biochem. 51, 44-55, 1973 |
| | | m24s3 | 2.60E-03 | | M | | | | Can. J. Biochem. 51, 44-55, 1973 |
| | | m24p1 | 1.00E-04 | | M | | | | J. Biol. Chem. 245, 2758-2762, 1970 |
| | | m24p2 | 2.00E-05 | | M | | | | J. Biol. Chem. 245, 2758-2762, 1970 |
| | | m24p3 | 1.50E-06 | | M | | | | J. Biol. Chem. 245, 2758-2762, 1970 |
| SDH r25 | revuumm | k25f | 5.10E+03 | | min⁻¹ | SUCC Q | FUM QH2 | | Arch. Biochem. Biophys. 369, 223-232, 1999 |
| | | k25r | 1.02E+02 | | min⁻¹ | | | | Arch. Biochem. Biophys. 369, 223-232, 1999 |
| | | m25s | 2.00E-06 | | M | | | | Arch. Biochem. Biophys. 369, 223-232, 1999 |
| | | m25p | 5.00E-06 | | M | | | | Arch. Biochem. Biophys. 369, 223-232, 1999 |
| FRD r26 | revuumm | k26f | 8.40E+02 | | min⁻¹ | SUCC FAD | FUM FADH | | Arch. Biochem. Biophys. 369, 223-232, 1999 |
| | | k26r | 1.06E+04 | | min⁻¹ | | | | Arch. Biochem. Biophys. 369, 223-232, 1999 |
| | | m26s | 1.50E-06 | | M | | | | Arch. Biochem. Biophys. 369, 223-232, 1999 |
| | | m26p | 5.40E-06 | | M | | | | Arch. Biochem. Biophys. 369, 223-232, 1999 |

Table 4 (continued)

| Enzyme | Type | Parameter | Value | fold-change | Unit | Substrate | Product | Effector | Reference |
|---|---|---|---|---|---|---|---|---|---|
| FUMA | revuumm | k27f | 1.86E+05 | | min⁻¹ | FUM | MAL | | Arch. Biochem. Biophys. 311, 509-516, 1994 |
| r27 | | k27r | 4.02E+04 | | min⁻¹ | | | | Arch. Biochem. Biophys. 311, 509-516, 1994 |
| | | m27s | 1.60E-05 | | M | | | | Arch. Biochem. Biophys. 311, 509-516, 1994 |
| | | m27p | 1.97E-02 | | M | | | | Arch. Biochem. Biophys. 311, 509-516, 1994 |
| MDH | revbbmm | k28f | 1.26E+03 | 20 | min⁻¹ | MAL | OAA | | Arch. Biochem. Biophys. 382, 15-21, 2000 |
| r28 | | k28r | 5.40E+04 | | min⁻¹ | NAD | NADH | | Arch. Biochem. Biophys. 382, 15-21, 2000 |
| | | m28s1 | 2.60E-03 | | M | | | | Arch. Biochem. Biophys. 382, 15-21, 2000 |
| | | m28s2 | 2.60E-04 | | M | | | | Arch. Biochem. Biophys. 382, 15-21, 2000 |
| | | m28p1 | 4.90E-05 | | M | | | | Arch. Biochem. Biophys. 382, 15-21, 2000 |
| | | m28p2 | 6.10E-05 | | M | | | | Arch. Biochem. Biophys. 382, 15-21, 2000 |
| FBP | noncompunimm | k29f | 8.76E+02 | | min⁻¹ | FDP | F6P | AMP | Biochim. Biophys. Acta 1594, 6-16, 2002 |
| r29 | | m29f | 1.54E-05 | | M | | Pi | | Biochim. Biophys. Acta 1594, 6-16, 2002 |
| | | k29i | 2.70E-06 | | M | | | | Biochim. Biophys. Acta 1594, 6-16, 2002 |
| PPS | revbtmm | k30f | 1.75E+06 | | min⁻¹ | PYR | PEP | | J. Biol. Chem. 245, 5309-5318, 1979 |
| r30 | | k30r | 1.33E+05 | | min⁻¹ | ATP | AMP | | J. Biol. Chem. 245, 5309-5318, 1979 |
| | | m30s1 | 8.30E-05 | | M | | Pi | | J. Biol. Chem. 245, 5309-5318, 1979 |
| | | m30s2 | 2.80E-05 | | M | | | | J. Biol. Chem. 245, 5309-5318, 1979 |
| | | m30p1 | 3.70E-05 | | M | | | | Methods Enzymol. 13, 309-314, 1969 |
| | | m30p2 | 1.10E-04 | | M | | | | Methods Enzymol. 13, 309-314, 1969 |
| | | m30p3 | 3.80E-02 | | M | | | | Methods Enzymol. 13, 309-314, 1969 |
| PPC | noncompbimm2 | k31f | 9.00E+03 | | min⁻¹ | PEP | OAA | ASP | Proc. Natl. Acad. Sci. USA 96, 823-828, 1999 |
| r31 | | m31s1 | 1.90E-04 | | M | CO2 | PI | MAL | Proc. Natl. Acad. Sci. USA 96, 823-828, 1999 |
| | | m31s2 | 1.00E-04 | | M | | | | Proc. Natl. Acad. Sci. USA 96, 823-828, 1999 |
| | | k31i1 | 2.00E-03 | | M | | | | Eur. J. Biochem. 247, 74-81, 1997 |
| | | k31i2 | 9.00E-04 | | M | | | | Eur. J. Biochem. 247, 74-81, 1997 |
| NADME | csmm | k32f | 3.54E+04 | | min⁻¹ | MAL | PYR | | J. Biochem. 76, 1259-1268, 1974 |
| r32 | | m32s1 | 1.90E-04 | | M | NAD | NADH | | J. Biochem. 76, 1259-1268, 1974 |
| | | m32s2 | 4.60E-05 | | M | | CO2 | | J. Biochem. 76, 1259-1268, 1974 |
| | | n32 | 1.00E+00 | | | | | | J. Biochem. 76, 1259-1268, 1974 |
| NADPME | csmm | k33f | 4.47E+04 | | min⁻¹ | MAL | PYR | | J. Biochem. 85, 1355-1365, 1979 |
| r33 | | m33s1 | 5.60E-03 | | M | NADP | NADPH | | Biochemistry 20, 2503-2512, 1981 |
| | | m33s2 | 1.50E-05 | | M | | CO2 | | Biochemistry 20, 2503-2512, 1981 |
| | | n33 | 1.00E+00 | | | | | | Biochemistry 20, 2503-2512, 1981 |

Table 4 (continued)

| Enzyme | Type | Parameter | Value | fold-change | Unit | Substrate | Product | Effector | Reference |
|---|---|---|---|---|---|---|---|---|---|
| PCK r34 | revbtmm | k34f | 2.53E+02 | 20 | min$^{-1}$ | OAA | PEP | | J. Biol. Chem. 255, 1399-1405, 1980 |
| | | k34r | 9.20E-02 | | min$^{-1}$ | ATP | ADP | | Can. J. Biochem. 58, 309-318, 1980 |
| | | m34s1 | 6.70E-04 | | M | | CO2 | | Can. J. Biochem. 58, 309-318, 1980 |
| | | m34s2 | 6.00E-05 | | M | | | | Can. J. Biochem. 58, 309-318, 1980 |
| | | m34p1 | 7.00E-05 | | M | | | | Can. J. Biochem. 58, 309-318, 1980 |
| | | m34p2 | 5.00E-05 | | M | | | | Can. J. Biochem. 58, 309-318, 1980 |
| | | m34p3 | 1.30E-02 | | M | | | | Can. J. Biochem. 58, 309-318, 1980 |
| ICL r35 | uscimm | k35f | 9.50E+03 | | min$^{-1}$ | ICIT | SUCC | 3PG | Biochem. J. 250, 25-31, 1988 |
| | | m35s | 6.30E-05 | | M | | GLX | | Biochem. J. 250, 25-31, 1988 |
| | | m35i | 8.00E-04 | | M | | | | Biochem. J. 250, 25-31, 1988 |
| MSA r36 | csmm | k36f | 3.00E+03 | 5 | min$^{-1}$ | ACCoA | MAL | OAA | Microbiology 140, 3099-3108, 1994 |
| | | m36s1 | 1.20E-05 | | min$^{-1}$ | GLX | CoA | | Microbiology 140, 3099-3108, 1994 |
| | | m36s2 | 8.20E-05 | | M | | | | Biochim. Biophys. Acta 99, 246-258, 1965 |
| | | n36 | 1.00E+00 | | | | | | |
| ICDK r37 | noncompunimm | k37f | 2.70E+02 | | min$^{-1}$ | ICDA | ICDA-P | 3PG | Eur. J. Biochem. 141, 401-408, 1984 |
| | | m37s1 | 3.50E-07 | | M | ATP | ADP | | Eur. J. Biochem. 141, 409-412, 1984 |
| | | m37s2 | 8.80E-05 | | M | | | | Eur. J. Biochem. 141, 409-412, 1984 |
| | | m37i | 1.20E-03 | | M | | | | Nature 305, 286-290, 1983 |
| ICDP r38 | icdp | k38f | 1.90E+01 | 10 | min$^{-1}$ | ICDA-P | ICDA | 3PG | Eur. J. Biochem. 141, 401-408, 1984 |
| | | m38s | 2.60E-06 | | M | | Pi | | Nature 305, 286-290, 1983 |
| | | m38a | 3.10E-03 | | M | | | | Nature 305, 286-290, 1983 |
| | | b38 | 1.02E+01 | | | | | | Nature 305, 286-290, 1983 |
| CYA r39 | noncompunimm | k39f | 8.10E+02 | | min$^{-1}$ | ATP | cAMP | ATP | J. Biol. Chem. 258, 3750-3758, 1983 |
| | | m39s | 1.00E-03 | | M | | | | J. Biol. Chem. 258, 3750-3758, 1983 |
| | | k39i | 1.40E-03 | | M | | | | J. Biol. Chem. 258, 3750-3758, 1983 |
| CYAA r39a | noncompunimm | KeqCYAact | 2.75E+03 | | | ATP | cAMP | ATP | J. Bacteriol. 180, 732-736, 1998 |
| | | k39fa | 8.10E+03 | | min$^{-1}$ | | | | J. Bacteriol. 180, 732-736, 1998 |
| | | m39sa | 1.00E-03 | | M | | | | J. Biol. Chem. 258, 3750-3758, 1983 |
| | | k39ia | 1.40E-03 | | M | | | | J. Biol. Chem. 258, 3750-3758, 1983 |
| CPDA r40 | smm | k40f | 6.20E+01 | | min$^{-1}$ | cAMP | AMP | ATP | J. Biol. Chem. 271, 25423-25429, 1996 |
| | | m40s | 5.00E-04 | | M | | | | J. Biol. Chem. 271, 25423-25429, 1996 |
| CEX r41 | ma | k41f | 2.10E+00 | | min$^{-1}$ | cAMP | cAMPxt | | Proc Natl Acad Sci USA 72, 2300-2304, 1975 |
| FRUK r42 | revbbmm | k42f | 1.01E+04 | | min$^{-1}$ | F1P | FDP | | Protein Expression and Purification 19, 48-52, 2000 |
| | | k42r | 5.00E+03 | | min$^{-1}$ | ATP | ADP | | Protein Expression and Purification 19, 48-52, 2000 |
| | | m42s1 | 1.25E-04 | | M | | | | Protein Expression and Purification 19, 48-52, 2000 |
| | | m42s2 | 6.00E-04 | | M | | | | Protein Expression and Purification 19, 48-52, 2000 |
| | | m42p1 | 5.00E-03 | | M | | | | |
| | | m42p2 | 5.00E-04 | | M | | | | |

## Table 5: Enzymatic reaction formulas

| | |
|---|---|
| fbmm | $$v = \frac{k_f[E][S]}{K_{mS}+[S]} - \frac{k_r[E-P][P]}{K_{mP}+[P]}$$ |
| ma2 | $$v = k_f[E_1 P][E_2] - k_r[E_1][E_2 P]$$ |
| smm | $$v = \frac{k_f[E][S]}{K_{mS}+[S]}$$ |
| revuumm | $$v = \frac{[E](k_f[S]/K_{mS} - k_r[P]/K_{mP})}{1+[S]/K_{mS}+[P]/K_{mP}}$$ |
| csmm | $$v = \frac{k[E]\left(\frac{[S_1]}{K_{mS1}}\right)^n \frac{[S_2]}{K_{mS2}}}{\left[1+\left(\frac{[S_1]}{K_{mS1}}\right)^n\right]\left(1+\frac{[S_2]}{K_{mS2}}\right)}$$ |
| ordub | $$v = \frac{k_f k_r[E]([S]-[P_1][P_2]/K_{eq})}{k_r K_{mS} + k_r[S] + k_f K_{mP2}[P_1]/K_{eq} + k_f K_{mP1}[P_2]/K_{eq} + k_r[S][P_2]/K_{iP1} + k_r[P_1][P_2]/K_{eq}}$$ |
| revtbmm | $$v = \frac{[E](k_f[S_1][S_2][S_3]/K_{mS1}K_{mS2}K_{mS3} - k_r[P_1][P_2]/K_{mP1}K_{mP2})}{[(1+[S_1]/K_{mS1})(1+[S_3]/K_{mS3})+[P_1]/K_{mP1}](1+[S_2]/K_{mS2}+[P_2]/K_{mP2})}$$ |
| revbbmm | $$v = \frac{[E](k_f[S_1][S_2]/K_{mS1}K_{mS2} - k_r[P_1][P_2]/K_{mP1}K_{mP2})}{(1+[S_1]/K_{mS1}+[P_1]/K_{mP1})(1+[S_2]/K_{mS2}+[P_2]/K_{mP2})}$$ |
| sbmm | $$v = \frac{k[E][S_1][S_2]}{([S_1]+K_{mS1})([S_2]+K_{mS2})}$$ |
| irrord | $$v = \frac{k[E][S_1][S_2]}{K_{iS1}K_{mS2} + K_{mS2}[S_1] + K_{mS1}[S_2] + [S_1][S_2]}$$ |
| random ter | $$v = \frac{k[E][P_1][P_2][P_3]}{C+CP_1[P_1]+CP_2[P_2]+CP_3[P_3]+K_{mP1}[P_2][P_3]+K_{mP2}[P_1][P_3]+K_{mP3}[P_1][P_2]+[P_1][P_2][P_3]}$$ |
| icdbt | $$v = irrord - random\,ter$$ |
| kgdhhccs | $$v = \frac{k[E]([S_1]+\alpha[S_1]^2/K_2)}{K_{mS1}+[S_1]+[S_1]^2/K_2}$$ |
| rebttmm | $$v = \frac{[E](k_f[S_1][S_2][S_3]/K_{mS1}K_{mS2}K_{mS3} - k_r[P_1][P_2][P_3]/K_{mP1}K_{mP2}K_{mP3})}{[(1+[S_1]/K_{mS1})(1+[S_3]/K_{mS3})+[P_1]/K_{mP1}](1+[P_2]/K_{mP2})(1+[P_3]/K_{mP3})+[S_2]/K_{mS2}]}$$ |
| noncompunimm | $$v = \frac{k[E]([S]/K_{mS})}{(1+[S]/K_{mS})(1+[A]/K_{ia})}$$ |
| revbtmm | $$v = \frac{[E](k_f[S_1][S_2]/K_{mS1}K_{mS2} - k_r[P_1][P_2][P_3]/K_{mP1}K_{mP2}K_{mP3})}{[(1+[P_1]/K_{mP1})(1+[P_3]/K_{mP3})+[S_1]/K_{mS1}](1+[P_2]/K_{mP2})+[S_2]/K_{mS2}]}$$ |
| noncompbimm2 | $$v = \frac{k[E][S_1][S_2]/K_{mS1}K_{mS2}}{(1+[S_1]/K_{mS1})(1+[S_2]/K_{mS2})(1+[I_1]/K_{I1})(1+[I_2]/K_{I2})}$$ |
| uscimm | $$v = \frac{k[E][S]}{K_{mS}(1+[I]/K_i)+[S]}$$ |

<3> Equilibirium reactions

The algebraic equations were reduced for solutions by assuming that equilibirium is established for the binding of transcription factors and effectors and activation of CYA. CRP is a transcription factor involved in catabolite suppression, and [CRP-cAMP]tot produced by equilibration of CRP and cAMP as an effector thereof was represented as a solution of the quadratic equation shown in the row of CRP1 in Table 6. $[CRP]^{tot}$ and $[cAMP]^{tot}$ represent the total concentrations of intracellular CRP and cAMP, respectively. About 200 binding sites on the genome are known for CRP, and it is necessary to consider that equilibirium is established for CRP-cAMP and these binding sites. Assuming that the dissociation constant for this, $K_{dCRP}$ is $4 \times 10^{-8}$ (M), the concentration of CRP-cAMP binding with the promoter on the genome can be considered a solution of the quadratic equation shown in the row of CRP2 in Table 6. It is known that Mlc suppresses a target gene in the absence of glucose, whereas it binds to non-phosphorylated $IICB^{Glc}$ in the presence of glucose. $[Mlc\text{-}IICB^{Glc}]$ which binds with $IICB^{Glc}$ and thus is inactivated was represented as a solution of the quadratic equation shown in the row of Mlc in Table 6. Cra is a transcription factor known as an activator/repressor of many sugar metabolism-related genes. It was assumed that the Cra concentration was constant. [Cra-F1P] binding to F1P, which is an effector thereof, was represented as a solution of the quadratic equation represented in the row of Cra in Table 6. The effector of PdhR, which is a repressor of the *aceEF* gene coding for PDH, is PYR (Quail and Guest, Mol. Microbiol., 15, 519-529, 1995), and [PdhR-PYR] which binds with PYR and is thus inactivated was represented as a solution of the quadratic equation shown in the row of PdhR in Table 6. It has been suggested by Cortay et al. that IclR is a transcription factor that suppresses expression of the glyoxylic acid pathway, and the effector thereof is PEP (Cortay et al., EMBO J., 10, 675-679, 1991). [IclR-PEP] which binds with PEP and thus is inactivated was represented as a solution of the quadratic equation shown in the row of PclR in Table 6. Activation of CYA by phosphorylated $IIA^{Glc}$ ($IIA^{Glc}$-P) is known. Although the detailed mechanism is unknown, modeling was performed by assuming that CYA and $IIA^{Glc}$-P bind to each other to form an activated CYA (CYAA). Based on the difference in CYA activity between a wild type strain and a strain deficient in *crr,* which codes for $IIA^{Glc}$ (Reddy and Kamireddi, J. Bacteriol., 180, 732-736, 1998), the dissociation constant of CYAA, $K_{dCYAA}$, was predicted as $1.34 \times 10^{-4}$ (M). Based on this, concentration of the activated CYAA [CYAA] produced by the equilibirium of CYA and $IIA^{Glc}$-P was represented as a solution of the quadratic equation shown in the row of CYA in Table 6.

Table 6: Equilibirium equations

| CRP1 | $$[CRP\text{-}cAMP]^{tot} = \left([CRP]^{tot} + [cAMP]^{tot} + K_{dcAMP}^{CRP} - \sqrt{\left([CRP]^{tot} + [cAMP]^{tot} + K_{dcAMP}^{CRP}\right)^2 - 4[CRP]^{tot}[cAMP]^{tot}}\right)\Big/2$$ |
|---|---|
| CRP2 | $$[P\text{-}CRP\text{-}cAMP] = \left([CRP\text{-}cAMP]^{tot} + [P^{CRP}]^{tot} + K_d^{CRP} - \sqrt{\left([CRP\text{-}cAMP]^{tot} + [P^{CRP}]^{tot} + K_d^{CRP}\right)^2 - 4[CRP\text{-}cAMP]^{tot}[P^{CRP}]^{tot}}\right)\Big/2$$ |
| Mlc | $$[Mlc\text{-}IICB^{Glc}] = \left([Mlc]^{tot} + [IICB^{Glc}]^{tot} + K_{dIICB}^{Mlc} - \sqrt{\left([Mlc]^{tot} + [IICB^{Glc}]^{tot} + K_{dIICB}^{Mlc}\right)^2 - 4[Mlc]^{tot}[IICB^{Glc}]^{tot}}\right)\Big/2$$ |
| Cra | $$[Cra\text{-}F1P] = \left([Cra]^{tot} + [F1P]^{tot} + K_{dF1P}^{Cra} - \sqrt{\left([Cra]^{tot} + [F1P]^{tot} + K_{dF1P}^{Cra}\right)^2 - 4[Cra]^{tot}[F1P]^{tot}}\right)\Big/2$$ |
| PdhR | $$[PdhR\text{-}PYR] = \left([PdhR]^{tot} + [PYR]^{tot} + K_{dPYR}^{PdhR} - \sqrt{\left([PdhR]^{tot} + [PYR]^{tot} + K_{dPYR}^{PdhR}\right)^2 - 4[PdhR]^{tot}[PYR]^{tot}}\right)\Big/2$$ |
| PclR | $$[IclR\text{-}PEP] = \left([IclR]^{tot} + [PEP]^{tot} + K_{dPEP}^{IclR} - \sqrt{\left([IclR]^{tot} + [PEP]^{tot} + K_{dPEP}^{IclR}\right)^2 - 4[IclR]^{tot}[PEP]^{tot}}\right)\Big/2$$ |
| CYA | $$[CYAA] = \left([CYA]^{tot} + [IIA^{Glc}\text{-}P]^{tot} + K_d^{CYAA} - \sqrt{\left([CYA]^{tot} + [IIA^{Glc}\text{-}P]^{tot} + K_d^{CYAA}\right)^2 - 4[CYA]^{tot}[IIA^{Glc}\text{-}P]^{tot}}\right)\Big/2$$ |

<4> Modeling of gene expression

As for the gene expression of *E. coli,* modeling was performed for the genes to which the transcription factors CRP, Cra, Mlc, PdhR, and IclR relate by referring to the EcoCyc database (Keseler et al., Nucleic Acids Res., 33, D334-D337, 2005). As for the transcription factors (TF) per se, modeling of expression was performed for CRP, Mlc, PdhR and IclR. A list of the equations of transcription and translation and parameters of the genes is shown in Table 7, and the equations used for the gene expression are shown in Table 8. [$mRNA_{gene}$] represents the concentration of mRNA to be transcribed, [$P_{gene}$] represents the concentration of a promoter for a gene, and [RNAP□$^D$] represents the concentration of RNA polymerase bound with □$^D$. The parameters $k_{gene}^{base}$, $k_{gene}^{TF}$ and $k_{gene}^{dRNA}$ are a baseline transcription rate constant, a transcription rate constant for TF-binding gene, and a decomposition constant of mRNA, respectively. [Protein] represents the concentration of translated protein, and [Ribosome] represents the ribosome concentration. The parameters $k_{trans}$ and $k_{deg}$ represent a translation rate constant and a proteolysis rate constant, respectively. The NoTF equation was used for a gene of which control is not known and a gene for which control is not considered, and TF1 was used for a gene to which one transcription factor relates. For the genes to which two transcription factors relate (*crp, mlc, ptsG, ptsHI, aceBAK*), equation was mentioned for each gene. If the concentrations of the translated proteins of the same operon are different, it is considered that such difference is due to reduction of transcription product or difference in transcription efficiency. Therefore, $T_{Protein}$ (translation efficiency coefficient of protein) was defined, and the TL1 equation was used. The *sdhCDAB-sucABCD* operon of *E. coli* contains *sdhCDAB* coding for SDH, and *sucABCD* composed of *sucAB* coding for the E1 and E2 subunits of the KGDH complex and *sucCD* coding for SCS (Cunningham and Guest, Microbiology, 144, 2113-2123, 1998). In consideration of the fact that the *sucABCD* operon is also transcribed from $P_{suc}$ besides $P_{sdh}$, and coefficients concerning the translation efficiency, $T_{KGDH}$ and $T_{scs}$, for the KGDH complex and SCS, respectively, it was described by using TL (KGDH) and TL (SCS). Because it was reported that the ratio of the products of the *aceBAK* operon, ICL, MSA and ICDKP, is 1:0.3:0.003 (Chung et al., J. Bacteriol., 175, 4572-4575, 1993), translation constants $T_{MSA}$ and $T_{ICDKP}$ were defined for the translation of MSA and ICDKP, respectively, and TL1 was used.

As concentration of a promoter, a value at $\mu$ of 0.01 (min)$^{-1}$ was estimated based on the $\mu$-dependent intracellular gene number data described by Bremer and Dennis (Escherichia coli and Salmonella: Cellular and Molecular Biology/ Second Edition (Neidhardt F.C., Ed., pp.1553-1569, American Society for Microbiology Press, Washington, D.C., 1996), and used as a constant. As for the number of binding sites on the genome of CRP, a value at $\mu$ of 0.01 (min)$^{-1}$ was calculated on the presumption that about 200 of the binding sites of CRP are uniformly distributed over the genome. As the rate constant of transcription, a value calculated so that it should give the literature value of the protein concentration or specific activity of enzyme as a constant value was used. When two or more transcription rate constants were required in control by a transcription factor, they were calculated by using data of transcription activity or protein concentration in a transcription factor-deficient strain. As the mRNA decomposition rate, if any experimental value is available from literature for a certain gene, it was used, or otherwise, measurement data based on the DNA microarray experiment of Selinger et al. (Genome Res., 13, 216-223, 2003) were used.

Table 7: Equations and parameters for transcription and translation
The values changed during the simulation are specified in the column of "fold-change".

| Gene | Module | Parameter | Value | fold-change | Unit | Reference |
|---|---|---|---|---|---|---|
| crp | TF2 (crp) | KdcAMPCRP | 1.00E-04 | | M | Biochim Biophys Acta 1547, 1-17, 2001 |
| | | Pcrpbindtot | 2.56E-06 | | M | Escherichia coli and Salmonella 97, 1553-1569, 1996 |
| | | KdCRP | 4.00E-08 | | M | |
| | | Pcrptot | 1.48E-08 | | M | Escherichia coli and Salmonella 97, 1553-1569, 1996 |
| | | Kdp1crpCRP | 4.50E-08 | | M | Biochemistry 35, 1162-1172, 1996 |
| | | Kdp2crpCRP | 3.70E-07 | | M | Biochemistry 35, 1162-1172, 1996 |
| | | kcrpbase | 2.92E+04 | | (Mmin)-1 | Mol. Microbiol. 10, 341-350, 1993 |
| | | kcrpCRPCRP | 5.39E+04 | | (Mmin)-1 | Mol. Microbiol. 10, 341-350, 1993 |
| | | kcrpdrna | 1.40E-01 | | min-1 | Genome Res. 13, 216-223, 2003 |
| mlc | TF2 (mlc) | KdIICBMlc | 1.00E-07 | | | EMBO J. 20, 491-498, 2001 |
| | | Pmlctot | 2.94E-09 | | M | Escherichia coli and Salmonella 97, 1553-1569, 1996 |
| | | KdmlcMlc | 2.00E-07 | | M | |
| | | KdmlcCRP | 1.00E-08 | | M | |
| | | kmlcbase | 2.43E+03 | | (Mmin)-1 | EMBO J. 20, 5344-5352, 2000 |
| | | kmlcCRP | 2.02E+02 | | (Mmin)-1 | EMBO J. 20, 5344-5352, 2000 |
| | | kmlcMlc | 1.79E+03 | | (Mmin)-1 | EMBO J. 20, 5344-5352, 2000 |
| | | kmlcdrna | 3.85E-01 | | min-1 | Genome Res. 13, 216-223, 2003 |
| cra | constant | KdF1PCra | 5.00E-06 | | M | |
| | | Cratot | 3.00E-07 | | M | |
| cyaA | TF1 | PcyaAtot | 1.58E-08 | | M | Escherichia coli and Salmonella 97, 1553-1569, 1996 |
| | | KdcyaAcrp | 2.00E-08 | | M | Mol. Gen. Genet. 253, 198-204, 1996 |
| | | kcyaAbase | 2.23E+02 | | (Mmin)-1 | J. Biol. Chem. 258, 3750-3758, 1983 |
| | | kcyaACRP | 9.13E+01 | | (Mmin)-1 | J. Bacteriol. 180, 732-736, 1998 |
| | | kcyaAdrna | 1.10E-01 | | min-1 | Genome Res. 13, 216-223, 2003 |
| cpdA | NoTF | PcpdAtot | 1.39E-08 | | M | Escherichia coli and Salmonella 97, 1553-1569, 1996 |
| | | kcpdAbase | 1.28E+04 | 5 | (Mmin)-1 | J. Bacteriol. 116, 857-866, 1973 |
| | | kcpdAdrna | 1.40E-01 | | min-1 | Genome Res. 13, 216-223, 2003 |
| ptsHI | TF2 (ptsHI) | PptsHItot | 1.23E-08 | | M | Escherichia coli and Salmonella 97, 1553-1569, 1996 |
| | | KdptsHIMlc | 5.00E-09 | | | |
| | | KdptsHICRP | 1.00E-08 | | | |
| | | kptsHP1base | 9.53E+04 | | (Mmin)-1 | Can. J. Biochem. Cell Biol. 61, 29-37, 1983 |
| | | kptsHP0CRP | 5.26E+05 | | (Mmin)-1 | Can. J. Biochem. Cell Biol. 61, 29-37, 1983 |
| | | kptsHP1CRP | 6.15E+04 | | (Mmin)-1 | Can. J. Biochem. Cell Biol. 61, 29-37, 1983 |
| | | kptsHdrna | 8.90E-02 | | min-1 | Genome Res. 13, 216-223, 2003 |
| EI | | TEI | 9.10E-02 | | M | Can. J. Biochem. Cell Biol. 61, 29-37, 1983 |

EP 1 752 899 A1

Table 7 (continued)

| Gene | Module | Parameter | Value | fold-change | Unit | Reference |
|---|---|---|---|---|---|---|
| *crr* | NoTF | Pcrrtot | 1.23E-08 | | M | Escherichia coli and Salmonella 97, 1553-1569, 1996 |
| | | kcrrP2 | 7.57E+04 | | (Mmin)-1 | J. Bacteriol. 148, 257-264, 1981 |
| | | kcrrdrna | 8.70E-02 | | min-1 | Genome Res. 13, 216-223, 2003 |
| *ptsG* | TF2 (ptsG) | PptsGtot | 1.32E-08 | | M | Escherichia coli and Salmonella 97, 1553-1569, 1996 |
| | | KdptsGMlc | 2.00E-09 | | | |
| | | KdptsGCRP | 2.00E-09 | | | |
| | | kptsGCRP | 2.57E+05 | 0.795 | (Mmin)-1 | Proc. Natl. Acad. Sci. USA. 84, 930-934, 1987 |
| | | kptsGdrna | 2.17E-01 | | min-1 | Genome Res. 13, 216-223, 2003 |
| *pgi* | NoTF | Ppgitot | 1.50E-08 | | M | Escherichia coli and Salmonella 97, 1553-1569, 1996 |
| | | kpgibase | 2.41E+04 | | (Mmin)-1 | Arch. Microbiol. 127 289-298, 1980 |
| | | kpgidrna | 1.24E-01 | | min-1 | Genome Res. 13, 216-223, 2003 |
| *pfkA* | TFI | PpfkAtot | 1.54E-08 | | M | Escherichia coli and Salmonella 97, 1553-1569, 1996 |
| | | KdpkfACra | 3.50E-09 | | M | Mol. Microbiol. 21, 257-26, 1996 |
| | | kpfkAbase | 3.68E+03 | 2.75 | (Mmin)-1 | J. Bacteriol. 171, 2424-2434, 1989 |
| | | kpfkACra | 1.37E+03 | 2.75 | (Mmin)-1 | J. Bacteriol. 171, 2424-2434, 1989 |
| | | kpfkAdrna | 9.90E-02 | | min-1 | Genome Res. 13, 216-223, 2003 |
| *fba* | NoTF | Pfbatot | 1.36E-08 | | | Escherichia coli and Salmonella 97, 1553-1569, 1996 |
| | | kfbabase | 2.50E+04 | | (Mmin)-1 | Biochemical. J. 169, 633-641, 1978 |
| | | kfbadrna | 6.50E-02 | | min-1 | Genome Res. 13, 216-223, 2003 |
| *tpiA* | NoTF | PtpiAtot | 1.54E-08 | | | *Escherichia coli* and *Salmonella* 97, 1553-1569 |
| | | ktpiAbase | 4.22E+04 | | (Mmin)-1 | J. Biol. Chem. 270, 29096-29104, 1995 |
| | | ktpiAdrna | 6.80E-02 | | min-1 | Genome Res. 13, 216-223, 2003 |
| *gapA* | NoTF | PgapAtot | 1.08E-08 | | M | Escherichia coli and Salmonella 97, 1553-1569, 1996 |
| | | kgapAbase | 6.09E+04 | | (Mmin)-1 | Biochem. J. 179, 99-107, 1979 |
| | | kgapAdrna | 1.16E-01 | | min-1 | J. Bacteriol. 176, 830-839, 1994 |
| *epd-pgk* | TFI | Pepdtot | 1.37E-08 | | M | Escherichia coli and Salmonella 97, 1553-1569, 1996 |
| | | KdepdCra | 5.00E-09 | | M | Mol. Microbiol. 21, 257-266, 1996 |
| | | kepdbase | 5.68E+04 | | (Mmin)-1 | J. Bacteriol. 178, 3411-3417, 1996 |
| | | kepdCra | 1.10E+04 | | (Mmin)-1 | J. Bacteriol. 178, 3411-3417, 1996 |
| | | kpgkdrna | 1.47E-01 | | min-1 | Genome Res. 13, 216-223, 2003 |
| *gpmA* | NoTF | PgpmAtot | 1.19E-08 | | M | Escherichia coli and Salmonella 97, 1553-1569, 1996 |
| | | kgpmAbase | 8.84E+04 | | (Mmin)-1 | FEBS Lett. 455, 344-348, 1999 |
| | | kgpmAdrna | 7.15E-02 | | min-1 | Genome Res. 13, 216-223, 2003 |
| *yibO* | NoTF | PyibOtot | 1.57E-08 | | M | Escherichia coli and Salmonella 97, 1553-1569, 1996 |
| | | kyibObase | 4.17E+04 | | (Mmin)-1 | FEBS Lett. 455, 344-348, 1999 |
| | | kyibOdrna | 1.93E-01 | | min-1 | Genome Res. 13, 216-223, 2003 |

Table 7 (continued)

| Gene | Module | Parameter | Value | fold-change | Unit | Reference |
|---|---|---|---|---|---|---|
| *eno* | NoTF | Penotot | 1.32E-08 | | M | Escherichia coli and Salmonella 97, 1553-1569, 1996 |
| | | kenobase | 8.93E+04 | | (Mmin)-1 | J. Biol. Chem. 246, 6797-6802, 1971 |
| | | kenodrna | 9.50E-02 | | min-1 | Biosci. Biotechnol. Biochem. 66, 2216-2220, 2002 |
| *pykF* | TF1 | PpykFtot | 1.26E-08 | | M | Escherichia coli and Salmonella 97, 1553-1569, 1996 |
| | | KdpykFcra | 4.00E-09 | | M | |
| | | kpykFbase | 8.14E+03 | 0.5 | (Mmin)-1 | J. Bacteriol. 171, 2424-2434, 1989 |
| | | kpykFCra | 2.01E+03 | 0.5 | (Mmin)-1 | J. Bacteriol. 171, 2424-2434, 1989 |
| | | kpykFdrna | 7.10E-02 | | min-1 | Genome Res. 13, 216-223, 2003 |
| *zwf* | NoTF | Pzwftot | 1.09E-08 | | M | Escherichia coli and Salmonella 97, 1553-1569, 1996 |
| | | kzwfbase | 3.37E+04 | | (Mmin)-1 | J. Bacteriol. 110, 155-160, 1972 |
| | | kzwfdrna | 2.31E-01 | | min-1 | J. Bacteriol. 173, 4660-4667, 1991 |
| *gnd* | NoTF | Pgndtot | 1.13E-08 | | (Mmin)-1 | Escherichia coli and Salmonella 97, 1553-1569, 1996 |
| | | kgndbase | 4.40E+04 | 2 | (Mmin)-1 | J. Bacteriol. 176, 115-122, 1994 |
| | | kgnddrna | 1.73E-01 | | min-1 | J. Bacteriol. 176, 115-122, 1994 |
| *rpiA* | NoTF | PrpiAtot | 1.36E-08 | | (Mmin)-1 | Escherichia coli and Salmonella 97, 1553-1569, 1996 |
| | | krpiAbase | 5.13E+03 | | (Mmin)-1 | J. Bacteriol. 175, 5628-5635, 1993 |
| | | krpiAdrna | 1.20E-01 | | min-1 | Genome Res. 13, 216-223, 2003 |
| *rpe* | NoTF | Prpetot | 1.49E-08 | | (Mmin)-1 | Escherichia coli and Salmonella 97, 1553-1569, 1996 |
| | | krpebase | 7.95E+03 | 2 | (Mmin)-1 | |
| | | krpedrna | 1.20E-01 | | min-1 | |
| *talB* | NoTF | PtalBtot | 1.39E-08 | | (Mmin)-1 | Escherichia coli and Salmonella 97, 1553-1569, 1996 |
| | | ktalBbase | 5.27E+03 | | (Mmin)-1 | J. Bacteriol. 177, 5930-5936, 1995 |
| | | ktalBdrna | 7.40E-02 | | min-1 | Genome Res. 13, 216-223, 2003 |
| tktA | NoTF | PtktAtot | 1.37E-08 | | (Mmin)-1 | *Escherichia coli* and *Salmonella* 97, 1553-1569 |
| | | ktktAbase | 3.34E+03 | | (Mmin)-1 | Eur. J. Biochem. 230, 525-532, 1995 |
| | | ktktAdrna | 8.00E-02 | | min-1 | Genome Res. 13, 216-223, 2003 |
| *pdhR-aceEF* | TF1 | KdPYRPdhR | 2.00E-05 | | | |
| | | PpdhRtot | 1.36E-08 | | (Mmin)-1 | Escherichia coli and Salmonella 97, 1553-1569, 1996 |
| | | KdPdhRpdhR | 5.00E-09 | | M | Mol. Microbiol. 15, 519-529, 1995 |
| | | kpdhRbase | 1.28E+03 | 0.0667 | (Mmin)-1 | Methods Enzymol. 89, 391-399, 1982 |
| | | kpdhRPdhR | 1.95E+02 | 0.0667 | (Mmin)-1 | Eur. J. Biochem. 20, 169-178, 1971 |
| | | kpdhRdrna | 8.90E-02 | | min-1 | Genome Res. 13, 216-223, 2003 |
| | | TPdhR | 2.00E-01 | | min-1 | |

# Table 7 (continued)

| Gene | Module | Parameter | Value | fold-change | Unit | Reference |
|---|---|---|---|---|---|---|
| gltA | NoTF | PgltAtot | 1.20E-08 | | (Mmin)-1 | Escherichia coli and Salmonella 97, 1553-1569, 1996 |
| | | kgltAbase | 2.27E+04 | 4 | (Mmin)-1 | J. Bacteriol. 176, 5086-5092, 1994 |
| | | kgltAdrna | 4.95E-01 | | min-1 | J. Bacteriol. 175, 5725-5727, 1993 |
| acnA | TF1 | PacnAtot | 1.07E-08 | | (Mmin)-1 | Escherichia coli and Salmonella 97, 1553-1569, 1996 |
| | | kacnAbase | 1.44E+03 | 1.3 | (Mmin)-1 | Microbiology 140, 2531-2541, 1994 |
| | | kacnACRP | 5.30E+03 | 1.3 | (Mmin)-1 | Microbiology 143, 3795-3805, 1997 |
| | | kacnAdrna | 7.80E-02 | | min-1 | Genome Res. 13, 216-223, 2003 |
| acnB | TF1 | PacnBtot | 1.35E-08 | | M | Escherichia coli and Salmonella 97, 1553-1569, 1996 |
| | | KdacnBCRP | 5.00E-08 | | M | |
| | | kacnBbase | 4.73E+03 | 1.4 | (Mmin)-1 | Microbiology 140, 2531-2541, 1994 |
| | | kacnBCRP | 1.33E+04 | 1.4 | (Mmin)-1 | Microbiology 143, 3795-3805, 1997 |
| | | kacnBdrna | 9.40E-02 | | min-1 | Genome Res. 13, 216-223, 2003 |
| icdA | TF1 | PicdAtot | 1.10E-08 | | M | Escherichia coli and Salmonella 97, 1553-1569, 1996 |
| | | KdicdACra | 1.00E-08 | | M | J. Bacteriol. 181, 893-898, 1999 |
| | | kicdAbase | 4.37E+04 | | (Mmin)-1 | J. Bacteriol. 171, 2424-2434, 1989 |
| | | kicdACra | 1.71E+05 | | (Mmin)-1 | J. Bacteriol. 171, 2424-2434, 1989 |
| | | kicdAdrna | 8.90E-02 | | min-1 | |
| sdhCDAB | NoTF | PsdhCtot | 1.20E-08 | | (Mmin)-1 | Escherichia coli and Salmonella 97, 1553-1569, 1996 |
| | | ksdhCDABbase | 1.04E+05 | | (Mmin)-1 | J. Bacteriol. 179, 4138-4142, 1997 |
| | | ksdhCDABdrna | 2.10E-01 | | min-1 | Microbiology 144, 2113-2123, 1998 |
| sucABCD | NoTF | Psuctot | 1.20E-08 | | (Mmin)-1 | Escherichia coli and Salmonella 97, 1553-1569, 1996 |
| | | ksucABCDbase | 1.23E+04 | 10 | (Mmin)-1 | J. Gen. Microbiol. 132, 1753-1762, 1986 |
| | | ksucABdrna | 1.93E-01 | | min-1 | Microbiology 144, 2113-2123, 1998 |
| KGDH | | TKGDH | 4.02E-02 | | | Methods Enzymol. 13, 55-61, 1969 |
| SCS | | TSCS | 6.92E-01 | | | J. Bacteriol. 179, 4138-4142, 1997 |
| frdABCD | NoTF | Pfrdtot | 1.46E-08 | | (Mmin)-1 | Escherichia coli and Salmonella 97, 1553-1569, 1996 |
| | | kfrdbase | 6.43E+03 | | (Mmin)-1 | Methods Enzymol. 126, 377-386, 1986 |
| | | kfrddrna | 1.17E-01 | | min-1 | Genome Res. 13, 216-223, 2003 |
| fumA | NoTF | PfumAtot | 1.04E-08 | | (Mmin)-1 | Escherichia coli and Salmonella 97, 1553-1569, 1996 |
| | | kfumAbase | 3.91E+04 | | (Mmin)-1 | J. Bacteriol. 183, 461-467, 2001 |
| | | kfumAdrna | 1.24E-01 | | min-1 | Genome Res. 13, 216-223, 2003 |
| mdh | NoTF | Pmdhtot | 1.45E-08 | | (Mmin)-1 | Escherichia coli and Salmonella 97, 1553-1569, 1996 |
| | | kmdhbase | 1.44E+04 | | (Mmin)-1 | J. Bacteriol. 163, 1074-1079, 1985 |
| | | kmdhdrna | 8.90E-02 | | min-1 | |
| fbp | TF1 | Pfbptot | 1.44E-08 | | (Mmin)-1 | Escherichia coli and Salmonella 97, 1553-1569, 1996 |
| | | KdfbpCra | 5.00E-07 | | M | |
| | | kfbpbase | 2.58E+02 | | (Mmin)-1 | Arch. Biochem. Biophys. 225, 944-949, 1983 |
| | | kfbpCra | 2.62E+03 | | (Mmin)-1 | J. Bacteriol. 171, 2424-2434, 1989 |
| | | kfbpdrna | 8.90E-02 | | min-1 | |

Table 7 (continued)

| Gene | Module | Parameter | Value | fold-change | Unit | Reference |
|---|---|---|---|---|---|---|
| *ppsA* | TF1 | PppsAtot | 1.06E-08 | | (Mmin)-1 | Escherichia coli and Salmonella 97, 1553-1569, 1996 |
| | | KdppsACra | 1.00E-07 | . | M | |
| | | kppsAbase | 2.06E+03 | 0.02 | (Mmin)-1 | J. Biol. Chem. 245, 5309-5318, 1979 |
| | | kppsACra | 9.45E+04 | 0.02 | (Mmin)-1 | J. Bacteriol. 171, 2424-2434, 1989 |
| | | kppsAdrna | 6.70E-02 | | min-1 | Genome Res. 13, 216-223, 2003 |
| *ppc* | NoTF | Pppctot | 1.53E-08 | | (Mmin)-1 | Escherichia coli and Salmonella 97, 1553-1569, 1996 |
| | | kppcbase | 2.30E+03 | 10 | (Mmin)-1 | J. Biol. Chem. 247, 5785-5792, 1972 |
| | | kppcdrna | 1.17E-01 | | min-1 | Genome Res. 13, 216-223, 2003 |
| *sfcA* | NoTF | PsfcAtot | 1.03E-08 | | (Mmin)-1 | Escherichia coli and Salmonella 97, 1553-1569, 1996 |
| | | ksfcAbase | 4.51E+02 | 0.2 | (Mmin)-1 | J. Biochem. 72, 1015-1027, 1972 |
| | | ksfcAdrna | 1.05E-01 | | min-1 | Genome Res. 13, 216-223, 2003 |
| *b2463* | NoTF | Pb2463tot | 1.24E-08 | | (Mmin)-1 | Escherichia coli and Salmonella 97, 1553-1569, 1996 |
| | | kb2463base | 2.30E+02 | 0.2 | (Mmin)-1 | J. Biochem. 72, 1015-1027, 1972 |
| | | kb2463drna | 9.40E-02 | | min-1 | Genome Res. 13, 216-223, 2003 |
| *pckA* | TF1 | PpckAtot | 1.49E-08 | | (Mmin)-1 | Escherichia coli and Salmonella 97, 1553-1569, 1996 |
| | | KdpckACra | 1.00E-07 | | M | |
| | | kpckAbase | 1.88E+02 | 2 | (Mmin)-1 | J. Biol. Chem. 255, 1399-1405, 1980 |
| | | kpckACra | 1.68E+03 | 2 | (Mmin)-1 | J. Bacteriol. 171, 2424-2434, 1989 |
| | | kpckAdrna | 6.70E-02 | | min-1 | Genome Res. 13, 216-223, 2003 |
| *iclR* | TF1 | KdPEPIclR | 5.00E-04 | | M | |
| | | PiclRtot | 1.51E+00 | | M | Escherichia coli and Salmonella 97, 1553-1569, 1996 |
| | | KdiclRIclR | 1.00E-09 | | M | Mol. Microbiol. 47, 183-194, 2003 |
| | | iclRbase | 1.18E+03 | | (Mmin)-1 | |
| | | kiclRIclR | 1.62E+02 | | (Mmin)-1 | J. Bacteriol. 178, 321-324, 1996 |
| | | kiclRdrna | 2.10E-01 | | min-1 | |
| *aceBAK* | TF2 (aceBAK) | PaceBKAtot | 1.51E-08 | | (Mmin)-1 | Escherichia coli and Salmonella 97, 1553-1569, 1996 |
| | | KdaceBAKiclR | 1.00E-09 | | M | Mol. Microbiol. 47, 183-194, 2003 |
| | | KdaceBAKCra | 3.00E-09 | | M | J. Mol. Biol. 234, 28-44, 1993 |
| | | kaceBAKbase | 2.52E+05 | 4 | (Mmin)-1 | J. Bacteriol. 171, 2424-2434, 1989 |
| | | kaceBAKCra | 4.66E+04 | 4 | (Mmin)-1 | J. Bacteriol. 172, 2642-2649, 1990 |
| | | kaceBAKdrna | 2.30E-01 | | min-1 | Genome Res. 13, 216-223, 2003 |
| MSA | | TMSA | 3.00E-01 | | | J. Bacteriol. 175, 4572-4575, 1993 |
| ICDKP | | TICDKP | 3.00E-03 | | | J. Bacteriol. 175, 4572-4575, 1993 |
| *fruBKA* | TF1 | PfruBKAtot | 1.17E-08 | | (Mmin)-1 | Escherichia coli and Salmonella 97, 1553-1569, 1996 |
| | | KdfruBKACra | 1.00E-09 | | M | |
| | | kfruBKAbase | 4.09E+05 | | (Mmin)-1 | J. Biol. Chem. 245, 5309-5318, 1979 |
| | | kfruBKACra | 1.51E+04 | | (Mmin)-1 | J. Bacteriol. 171, 2424-2434, 1989 |
| | | kfruBKAdrna | 2.10E-01 | | min-1 | Genome Res. 13, 216-223, 2003 |

Table 8: Gene expression equations

| NoTF | $\dfrac{d[mRNA_{gene}]}{dt} = k_{gene}^{base}[RNAP\sigma^D][P_{gene}]^{tot} - (k_{gene}^{dRNA} + \mu)[mRNA_{gene}]$ |
|---|---|
| TF1 | $\dfrac{d[mRNA_{gene}]}{dt} = \dfrac{k_{gene}^{base} + k_{gene}^{Cra}[TF]/K_{dgene}^{Cra}}{1 + [TF]/K_{dgene}^{Cra}}[RNAP\sigma^D][P_{gene}]^{tot} - (k_{gene}^{dRNA} + \mu)[mRNA_{gene}]$ |
| TF2 (crp) | $\dfrac{d[mRNA_{crp}]}{dt} = \dfrac{k_{crp}^{base} + k_{mlc}^{CRP}[CRP\text{-}cAMP]/K_{dmlc}^{CRP} + k_{mlc}^{Mlc}\left([Mlc]/K_{dmlc}^{Mlc} + [CRP\text{-}cAMP][Mlc]/K_{dmlc}^{CRP}K_{dmlc}^{Mlc}\right)}{1 + [CRP\text{-}cAMP]/K_{dmlc}^{CRP} + [Mlc]/K_{dmlc}^{Mlc} + [CRP\text{-}cAMP][Mlc]/K_{dmlc}^{CRP}K_{dmlc}^{Mlc}}[RNAP\sigma^D][P_{crp}]^{tot} - (k_{crp}^{dRNA} + \mu)[mRNA_{crp}]$ |
| TF2 (mlc) | $\dfrac{d[mRNA_{mlc}]}{dt} = \dfrac{k_{mlc}^{base} + k_{mlc}^{CRP}[CRP\text{-}cAMP]/K_{dmlc}^{CRP} + k_{mlc}^{Mlc}\left([Mlc]/K_{dmlc}^{Mlc} + [CRP\text{-}cAMP][Mlc]/K_{dmlc}^{CRP}K_{dmlc}^{Mlc}\right)}{1 + [CRP\text{-}cAMP]/K_{dmlc}^{CRP} + [Mlc]/K_{dmlc}^{Mlc} + [CRP\text{-}cAMP][Mlc]/K_{dmlc}^{CRP}K_{dmlc}^{Mlc}}[RNAP\sigma^D][P_{crp}]^{tot} - (k_{mlc}^{dRNA} + \mu)[mRNA_{mlc}]$ |
| TF2 (ptsG) | $\dfrac{d[mRNA_{ptsG}]}{dt} = \dfrac{k_{ptsG}^{CRP}[CRP\text{-}cAMP]/K_{dptsG}^{CRP}}{1 + [CRP\text{-}cAMP]/K_{dptsG}^{CRP} + [Mlc]/K_{dptsG}^{Mlc} + [CRP\text{-}cAMP][Mlc]/K_{dptsG}^{CRP}K_{dptsG}^{Mlc}}[RNAP\sigma^D][P_{ptsG}]^{tot} - (k_{ptsG}^{dRNA} + \mu)[mRNA_{ptsG}]$ |
| TF2 (ptsHI) | $\dfrac{d[mRNA_{ptsHI}]}{dt} = \dfrac{k_{ptsHIP}^{base}\left(1 + [Mlc]/K_{dptsHI}^{mlc}\right) + k_{ptsHIPO}^{CRP}[CRP\text{-}cAMP]/K_{dptsHI}^{CRP} + k_{ptsHI}^{CRP}\left([CRP\text{-}cAMP]/K_{dptsHI}^{CRP} + [CRP\text{-}cAMP][Mlc]/K_{dptsHI}^{CRP}K_{dptsHI}^{mlc}\right)}{1 + [CRP\text{-}cAMP]/K_{dptsHI}^{CRP} + [Mlc]/K_{dptsHI}^{Mlc} + [CRP\text{-}cAMP][Mlc]/K_{dptsHI}^{CRP}K_{dptsHI}^{Mlc}}[RNAP\sigma^D][P_{ptsHI}]^{tot} - (k_{ptsHI}^{dRNA} + \mu)[mRNA_{ptsHI}]$ |
| TF2 (aceBAK) | $\dfrac{d[mRNA_{aceBAK}]}{dt} = \dfrac{k_{aceBAK}^{base} + k_{aceBAK}^{Cra}[Cra]/K_{daceBAK}^{Cra}}{1 + [Cra]/K_{daceBAK}^{Cra} + [IclR]/K_{daceBAK}^{IclR} + [Cra][IclR]/K_{daceBAK}^{Cra}K_{daceBAK}^{IclR}}[RNAP\sigma^D][P_{gene}]^{tot} - (k_{gene}^{dRNA} + \mu)[mRNA_{gene}]$ |
| TL | $\dfrac{d[Protein]}{dt} = k^{trans}[Ribosome][mRNA_{gene}] - (k^{deg} + \mu)[Protein]$ |
| TL1 | $\dfrac{d[Protein]}{dt} = k^{trans}[Ribosome][mRNA_{gene}]T_{protein} - (k^{deg} + \mu)[Protein]$ |
| TL (IIA$^{Glc}$) | $\dfrac{d[IIA^{Glc}]}{dt} = k^{trans}[Ribosome]\left([mRNA_{crr}] + [mRNA_{ptsHI}]T_{EI}\right) - (k^{deg} + \mu)[EI]$ |
| TL (KGDH) | $\dfrac{d[KGDH]}{dt} = k^{trans}[Ribosome]\left([mRNA_{sucABCD}] + [mRNA_{sdhCDAB}]\right)T_{KGDH} - (k^{deg} + \mu)[KGDH]$ |
| TL (SCS) | $\dfrac{d[SCS]}{dt} = k^{trans}[Ribosome]\left([mRNA_{sucABCD}] + [mRNA_{sdhCDAB}]\right)T_{SCS} - (k^{deg} + \mu)[SCS]$ |

<5> Preparation of mathematical equation for specific growth rate $\mu$ and cell formation rate

The specific growth rate $\mu$ is an index often used for representing growth. In order to represent growth with $\mu$ as accurately as possible, the following approximate equation of OD was obtained from OD data over time from culture of a wild type strain in a S-type jar by using a curve fitting program, TableCurve 2D (Systat Software), and a time function of $\mu$ was obtained from an equation obtained by differentiating the approximate equation. The result of plotting for OD and $\mu$ based on the approximate equation is shown in Fig. 2.

$$OD = (2.05 + 1.53 \times 10^{-5}t^2 - 5.35 \times 10^{-10}t^4 + 3.07 \times 10^{-25}t^6)/(1 - 1.76 \times 10^{-5}t^2 + 1.17 \times 10^{-10}t^4 - 3.19 \times 10^{-16}t^6 + 4.19 \times 10^{-22}t^8)$$

$$\mu = dOD/dt/OD$$

In order to compute the cell formation rate during the growth, metabolic reactions of E. *coli* were defined based on the report of Chassagnole et al. (Biotechnol. Bioeng., 79, 53-73, 2002). Synthetic reactions of the cell components were defined for each of protein synthesis, RNA synthesis, DNA replication, lipid synthesis, glycogen synthesis and peptidoglycan (murein) synthesis, and stoichiometric equations were defined from ratios of components (Neidhardt and Umbarger, Escherichia coli and Salmonella: Cellular and Molecular Biology (Neidhardt, F.C. Ed., pp.13-16, American Society for Microbiology and Washington D.C., 1996; Pramanik and Keasling, Biotechnol. Bioeng., 56, 398-421, 1997) and energy required for synthesis (Stephanopoulos et al., Metabolic Engineering: Principles and Methodologies, Academic Press, San Diego, 1998). Furthermore, a stoichiometric equation was prepared for each component required for synthesis of 1 g of cells from the composition of cells (Chassagnole et al., Biotechnol. Bioeng., 79, 53-73, 2002). This equation concerning the cell formation was converted into a stoichiometric equation using intermediate metabolites used in the simulation to create the following stoichiometric equation for each intermediate metabolite required for producing 1 g of cells.

$$
\begin{aligned}
\text{g\_biomass} = &\ 3.962\ \text{Pyr} + 1.229\ \text{aKG} + -2.232\ \text{CO}_2 + 10.91\ \text{NH}_4 \\
&+ 44.69\ \text{ATP} + -44.6\ \text{ADP} + -15.18\ \text{P} + 16.09\ \text{H} + 18.17\ \text{NADPH} \\
&+ -18.17\ \text{NADP} + 2.409\ \text{AcCoA} + -2.949\ \text{CoA} + -0.487\ \text{Fum} + \\
&2.393\ \text{OAA} + 1.957\ \text{3PG} + 0.252\ \text{SO4} + -2.329\ \text{NADH} + 2.329\ \text{NAD} \\
&+ 0.5402\ \text{SucCoA} + -0.4727\ \text{Suc} + 0.6887\ \text{PEP} + 0.3312\ \text{E4P} + \\
&0.4133\ \text{DHAP} + 0.1023\ \text{O2} + -0.0432\ \text{GAP} + 0.5312\ \text{R5P} + 0.1025 \\
&\text{F6P}
\end{aligned}
$$

The amount of the intermediate metabolite required for formation of 1 g of cells was converted into a value per cell volume and minute and incorporated into the differential equations as an equation of the specific growth rate $\mu$.

<6> Preparation of mathematical equations of RNA polymerase and ribosome

Transcription and translation in gene expression are catalyzed by RNA polymerase and ribosome, respectively. It is known that the molecular numbers of these enzymes change during the process of growth. From the data of $\mu$-dependent intracellular molecular number described by Bremer and Dennis (Escherichia coli and Salmonella: Cellular and Molecular Biology/Second Edition (Neidhardt F.C. Ed., pp.1553-1569, American Society for Microbiology Press, Washington, D.C., 1996), mathematical equations were prepared by using approximate equations. RNA polymerase binds with the $\square$ factor to become a holoenzyme and then function. Because $\square^D$ responsible for gene expression during the growth phase is substantially constant during the growing process, $\square^D$-bound RNA polymerase concentration [RNAP$\square^D$] was considered to be 1/3 of the total RNA polymerase concentration, and represented by the following equation.

$$[\text{RNAP}\square^D] = 6.67 \times 10^{-7} + 3.0 \times 10^{-4}\mu + 2.64 \times 10^{-2}\mu^2$$

As for ribosome, by fitting the data of Bremer and Dennis (Escherichia coli and Salmonella: Cellular and Molecular Biology/Second Edition (Neidhardt F.C. Ed., pp. 1553-1569, American Society for Microbiology Press, Washington, D.C., 1996) using TableCurve 2D, the following equation was obtained.

$$[\text{Ribosome}] = 1.90 \times 10^{-5} + 1.38\mu^2 + 10.2\mu^{2.5} + 36.6\mu^3$$

EP 1 752 899 A1

<7> Excretion to outside of cells and uptake from outside of cells

Among the substances excreted to the outside of cells, excretion of acetic acid (AcOH) and formic acid (Formate), of which amounts detected in culture of a wild type strain were large, was incorporated. Based on the measured data for extracellular concentrations of acetic acid and formic acid over time, the profiles over time were approximated to time functions, and the functions were converted into rates by differentiation and incorporated into the differential equations of ACCoA and PYR. The plot of the amount of acetic acid based on the approximated function and the rate obtained by differentiating the approximated function is shown in Fig. 4.

$$AcOH_{ex} = (2.49 \times 10^{-3} - 7.61 \times 10^{-3}t - 3.38 \times 10^{-7}t^2 + 9.33 \times 10^{-10}t^3)/(1 - 7.61 \times 10^{-3}t + 2.44 \times 10^{-5}t^2 - 1.05 \times 10^{-8}t^3)$$

$$Form_{ex} = 4.41 \times 10^{-4} - 1.17 \times 10^{-9}t^2 + 1.97 \times 10^{-13}t^4 + 3.93 \times 10^{-19}t^6$$

Among the organic substances existing in medium, amino acids and so forth are taken up into the cells. Uptake of glutamic acid (Glu) and alanine (Ala), of which existing amounts in culture of a wild type strain were large, was represented with mathematical equations and incorporated. Uptake of glutamic acid and alanine contained in the initial medium was approximated with the following time functions, and the functions were converted into rates by differentiation of the functions and incorporated into the differential equations of AKG and PYR.

$$Glu_{in} = 9.2 \times 10^{-4} - 7.26 \times 10^{-6}t$$

$$Ala_{in} = 8.36 \times 10^{-4} - 6.69 \times 10^{-6}t$$

<8> Culture of wild type strain MG1655 and metabolic flux analysis

The wild type strain MG1655 was cultured overnight in 30 ml of LB medium, and the cell were collected from the culture broth. The cells were cultured in MS medium using $^{13}$C glucose contained in an S-type jar under the conditions of batch culture. As for the composition of the MS medium, it had a composition of 40 g of glucose, 1 g of $MgSO_4 \cdot 7H_2O$, 16 g of $(NH_4)_2SO_4$, 1 g of $KH_2PO_4$, 2 g of Bacto yeast extract, 0.01 g of $MnSO_4 \cdot 4H_2O$, 0.01 g of $FeSO_4 \cdot 7H_2O$, and 0.5 ml of GD113 (antifoaming agent) in 1 L, and as for the culture conditions, the culture was carried out in a culture volume of 0.3 L, at a temperature of 37°C and pH 7.0 with aeration by stirring. Metabolic flux analysis was performed during the growth phase (315 minutes) and the stationary phase (495 minutes). The method of metabolic flux analysis is described in International Publication No. WO2005/001736 in detail. These culture results were used for verification of the simulation. Plots of the results of the measurements of extracellular glucose concentration and extracellular $CO_2$ concentration are shown in Fig. 8A, B and Fig. 8B, P, respectively (broken lines). Further, the results of the metabolic flux analysis during the growth phase (315 minutes) and the stationary phase (495 minutes) are shown in Table 9. The values of metabolic fluxes converted into enzymatic reaction rates are plotted to enzymatic activity (Fig. 9A, F and I, Fig. 9B, L, O and R).

Table 9: Results of metabolic flux analysis and conversion into enzymatic reaction rates

The metabolic fluxes are represented with values standardized in mmol/10 mmol Glc. The enzymatic activity is represented with values obtained by converting a sugar consumption rate at a corresponding time into actual enzymatic reaction rate (mol/min).

| Enzyme | Flux at 315 min (mmol/10mmol) | Rate at 315 min (M/min) | Flux at 495 min (mmol/10mmol) | Rate at 495 min (M/min) |
|---|---|---|---|---|
| IICBGlc | 10.00 | 0.0795 | 10.00 | 0.0497 |
| PGI | 6.02 | 0.0479 | 2.94 | 0.0146 |
| PFKA | 6.02 | 0.0478 | 7.05 | 0.0351 |
| TPIA | 6.02 | 0.0479 | 7.05 | 0.0351 |

33

(continued)

The metabolic fluxes are represented with values standardized in mmol/10 mmol Glc. The enzymatic activity is represented with values obtained by converting a sugar consumption rate at a corresponding time into actual enzymatic reaction rate (mol/min).

| Enzyme | Flux at 315 min (mmol/10mmol) | Rate at 315 min (M/min) | Flux at 495 min (mmol/10mmol) | Rate at 495 min (M/min) |
|---|---|---|---|---|
| GAPA | 16.47 | 0.1309 | 16.47 | 0.0819 |
| PGK | 16.47 | 0.1309 | 16.47 | 0.0819 |
| dGPM+iGPM | 14.58 | 0.1159 | 14.87 | 0.0739 |
| ENO | 14.58 | 0.1159 | 14.87 | 0.0739 |
| PYKF | 0.71 | 0.0057 | 2.52 | 0.0125 |
| PDH | 7.24 | 0.0575 | 10.46 | 0.0520 |
| G6PD | 3.98 | 0.0316 | 7.05 | 0.0351 |
| 6PGD | 3.98 | 0.0316 | 7.05 | 0.0351 |
| RPIA+RPE | 3.98 | 0.0316 | 7.05 | 0.0351 |
| TKTAI+TALB | 0.32 | 0.0026 | 0.22 | 0.0011 |
| TKTAII | 0.25 | 0.0020 | 1.62 | 0.0081 |
| CS | 4.02 | 0.0320 | 7.77 | 0.0386 |
| ACNA+ACNB | 4.02 | 0.0320 | 7.77 | 0.0386 |
| ICDA | 4.02 | 0.0320 | 7.21 | 0.0358 |
| KGDH | 2.87 | 0.0228 | 6.43 | 0.0320 |
| SCS | 2.87 | 0.0228 | 6.43 | 0.0320 |
| SDH | 93.10 | 0.7401 | 21.23 | 0.1056 |
| FRD | 90.00 | 0.7154 | 14.09 | 0.0701 |
| FUMA | 3.10 | 0.0246 | 7.14 | 0.0355 |
| MDH | 3.10 | 0.0246 | 7.14 | 0.0355 |
| FBA | 6.02 | 0.0479 | 7.05 | 0.0351 |
| PPSA | 0.00 | 0.0000 | 0.00 | 0.0000 |
| PCK | 0.01 | 0.0001 | 1.49 | 0.0074 |
| PPC | 2.81 | 0.0223 | 3.13 | 0.0155 |
| NADPME+NADME | 0.00 | 0.0000 | 0.30 | 0.0015 |
| ICL | 0.00 | 0.0000 | 0.56 | 0.0028 |
| MSA | 0.00 | 0.0000 | 0.56 | 0.0028 |

<9> Simulation and verification of E. *coli* central metabolic model

The simulation was performed by describing differential equations using a mathematical calculation program MATLAB (MathWorks) and using ode15s as an ODE solver. The differential equations of material balance used for the simulation are shown below. Material balance of each substance is described as the sum of enzymatic reaction rate, dilution effect due to growth, synthesis rate of cell component (y_biomass(metabolite)), uptake rate of extracellular substance and rate of excretion to the outside of cells mentioned in Table 4.

$$d[\text{Cellvoltot}]/dt = \mu * [\text{Cellvoltot}]$$

$$d[\text{Glucose}]/dt = - \text{rx1e}$$

$$d[\text{G6P}]/dt = \text{rx1e} - \text{rx2} - \text{rx12} - (\mu * [\text{G6P}])$$

```
d[F6P]/dt = rx2 + rx29 + rx16 + rx17b - rx3 - ( μ * [F6P])
- y_biomass(F6P) * mu / cellvol * cellweight            .


    d[FDP]/dt = rx3 - rx4 - rx29 - ( μ * [FDP]) -
    y_biomass(FDP) * μ / cellvol * cellweight


d[DHAP]/dt = rx4 - rx5 - ( μ * [DHAP]) - y_biomass(DHAP) *
μ / cellvol * cellweight


d[GA3P]/dt = rx4 + rx5 + rx17b - rx6 - rx16 - rx17 - ( μ *
[GA3P]) - y_biomass(GA3P) * μ / cellvol * cellweight


    d[13DPG]/dt = rx6 - rx7 - ( μ * [13DPG])


d[3PG]/dt = rx7 - rx8 - rx9 - ( μ * [3PG]) - y_biomass(3PG)
* μ / cellvol * cellweight


    d[2PG]/dt = rx8 + rx9 - rx10 - ( μ * [2PG])
    PEP


d[PEP]/dt = rx10 + rx30 + rx34 - rx11 - rx1a - rx31 - ( μ *
[PEP]) - y_biomass(11) * μ / cellvol * cellweight


d[PYR]/dt = rx11 + rx1a + rx32 + rx33 - rx18 - rx30 - ( μ *
[PYR]) - y_biomass(PYR) * mu / cellvol * cellweight +
Alauptake - Formin


    d[6PGC]/dt = rx12 - rx13 - ( μ * [6PGC])


    d[RL5P]/dt = rx13 - rx14 - rx15 - ( μ * [RL5P])


d[R5P]/dt = rx14 + rx17 - ( μ * [R5P]) - y_biomass(R5P) * μ
/ cellvol * cellweight
```

$$d[X5P]/dt = rx15 + rx17 - rx17b - (\mu * [X5P])$$

$$d[E4P]/dt = rx16 - rx17b - (\mu * [E4P]) - y\_biomass(E4P) * \mu / cellvol * cellweight$$

$$d[S7P]/dt = - rx16 - rx17 - (\mu * [S7P])$$

$$d[ACCoA]/dt = rx18 - rx19 - rx36 - (\mu * [ACCoA]) - AcOHin - y\_biomass(19) * mu / cellvol * cellweight + Formin$$

$$d[OAA]/dt = rx28 + rx31 - rx19 - rx34 - (\mu * [OAA]) - y\_biomass(OAA) * \mu / cellvol * cellweight$$

$$d[CIT]/dt = rx19 - rx20 - rx21 - (\mu * [CIT]) - y\_biomass(CIT) * \mu / cellvol * cellweight$$

$$d[ICIT]/dt = rx20 + rx21 - rx22 - rx35 - (\mu * [ICIT]) - y\_biomass(ICIT) * \mu / cellvol * cellweight$$

$$d[AKG]/dt = rx22 - rx23 - (\mu * [AKG]) - y\_biomass(AKG) * \mu / cellvol * cellweight + Gluuptake$$

$$d[SUCCoA]/dt = rx23 - rx24 - (\mu * [SUCCoA]) - y\_biomass(SUCCoA) * \mu / cellvol * cellweight$$

$$d[SUCC]/dt = rx24 + rx35 - rx25 - rx26 - (\mu * [SUCC]) - y\_biomass(SUCC) * \mu / cellvol * cellweight$$

$$d[FUM]/dt = rx25 + rx26 - rx27 - (\mu * [FUM]) - y\_biomass(FUM) * \mu / cellvol * cellweight$$

$$d[MAL]/dt = rx27 + rx36 - rx28 - rx32 - rx33 - (\mu * [MAL]) - y\_biomass(27) * \mu / cellvol * cellweight$$

$$d[GLX]/dt = rx35 - rx36 - (\mu * [GLX])$$

$$d[cAMP]/dt = rx39 + rx39a - rx40 - rx41 - (\mu * [cAMP])$$

$$d[cAMPex]/dt = rx41$$

$$d[F1P]/dt = - rx42 - (\mu * [F1P])$$

$$d[CO2]/dt = rx13 + rx18 + rx22 + rx23 + rx32 + rx33 + rx34$$
$$- rx31 - (\mu * [CO2]) - y\_biomass(32) * \mu / cellvol *$$
$$cellweight$$

During the simulation, a part of the parameters were manually changed to perform the simulation. The changed parameters are shown in Tables 3, 4 and 7. Among the results of the simulation of the *E. coli* central metabolic model, temporal changes of major metabolites are shown in Figs. 8A and 8B. In the process of growth, the extracellular glucose concentration (Fig. 8A, B) and the extracellular $CO_2$ concentration (Fig. 8B, P) showed behaviors extremely close to those of the measured values (broken lines), although deviation is seen for the first half ot the culture. Among the metabolites, metabolites other than PEP (Fig. 8A, F) showed temporal changes in the culture in the presence of glucose within the numerical ranges considered physiologically reasonable. The mRNA concentrations of major genes, the protein concentrations and the enzymatic activities are shown in Figs. 9A and 9B. Since all the initial values of mRNA concentrations were set at 0, they showed common patterns that they increased with increase in the growth rate, and decreased after $\mu$ reached the maximum (Fig. 9A, A etc.). The protein concentrations showed patterns that they decreased from the initial values, then increased with the increase in the growth rate, reached the maximum slightly behind the peak of mRNA, and decreased thereafter (Fig. 9A, B etc.). As for enzymatic activities, whereas a significant deviation from the results of the metabolic flux analysis was observed for the activity of the oxidative pentose phosphate pathway enzyme, G6PD (Fig. 9B, L), profiles close to those of the values of the enzymatic reaction rates based on the flux analysis data could be obtained for the activity of PFK in the glycolysis system (Fig. 9A, F), activity of the TCA cycle enzyme, CS (Fig. 9B, 0), and activity of PEPC of the supplemental pathway (Fig. 9B, R).

The results of simulation of gene expression where RNA polymerase and ribosome concentrations were independent from $\mu$ are shown in Figs. 10A and 10B. In this simulation, the values of the RNA polymerase and ribosome concentrations were those observed at $\mu$ of 0.01 $(min)^{-1}$, and only $k_{ptsG}^{CRP}$ among the parameters was changed to 0.45 time of the original value to perform the simulation. The results were physiologically unreasonable, for example, the mRNA level became constant (Fig. 10A, A etc.), the protein concentration increased in the second half of the culture where $\mu$ decreased (Fig. 10A, B etc.), and so forth. These results indicate that description of $\mu$-dependent RNA polymerase and ribosome concentrations is important for carrying out the simulation of the growing process. In order to investigate the effect of the cell formation accompanying the cell growth, simulation was performed with synthesis rates from intracellular metabolites to cell components of 0, and the results of the metabolic simulation are shown in Figs. 11A and 11B. It can be seen that many metabolites were accumulated in the cells, and the concentrations thereof are deviated from the physiological concentrations. Furthermore, in order to investigate effects of uptake and excretion of substances, simulation was performed with Glu and Ala uptake of 0 and acetic acid and formic acid excretion of 0, and the results of the metabolites are shown (Figs. 12A and 12B). It can be seen that evident differences were observed in the simulation results, such as those of AcCoA (Fig. 12B, J) and CIT (Fig. 12B, K), and they were deviated from the physiological concentrations. Thus, it was revealed that formation of cell components from intracellular metabolites, uptake and excretion of extracellular metabolites are necessary for realizing simulation similar to measurement results.

**Claims**

1. A method for effecting a simulation of a substance-production process that uses cells, wherein said simulation is based on a set of differential equations that represent intracellular metabolites and gene expression, said method comprising the steps of:

> (a) including a specific growth rate of cells, expressed as a differential equation, in the set of differential equations;
> (b) assigning values for parameters in the set of differential equations, wherein at least one of said parameters is represented as a growth rate factor;
> (c) incorporating in the set of differential equations a formation rate for formation of a cell component from an intracellular metabolite, wherein said formation rate is represented as a growth rate factor;
> (d) incorporating in the set of differential equations an inflow rate of a metabolite taken up from the outside of the cells and/or an outflow rate of a metabolite excreted out of the cells from the inside of the cells, wherein said inflow rate and said outflow rate are represented, respectively, as a growth rate factor;
> (e) solving the set of differential equations; and
> (f) generating data representative of the substance-production process.

2. The method according to claim 1, wherein the differential equations include the following equations (1) to (3):

$$d[Metabolite]/dt = V_{input} - V_{output} - \mu[Metabolite]$$

(Equation 1),

$$d[mRNA]/dt = k_{transcription}[P] - (k_{dRNA} + \mu)[mRNA]$$

(Equation 2),

and

$$d[Protein]/dt = k_{translation}[mRNA] - (k_{dProtein} + \mu)[Protein] \quad (Equation\ 3),$$

wherein, in the Equation 1, [Metabolite] represents an intracellular concentration of a metabolite, $V_{input}$ represents the sum of rates of reactions producing the metabolite, $V_{output}$ represents the sum of rates of reactions consuming the metabolite, and $\mu$ represents the specific growth rate;

in the Equation 2, [mRNA] represents a concentration of mRNA, $k_{transcription}$ represents a rate constant of transcription, [P] represents a promoter concentration, $k_{dRNA}$ represents a rate constant of decomposition of mRNA, and $\mu$ represents the specific growth rate, and

in the Equation 3, [Protein] represents a concentration of a protein, $k_{translation}$ represents a rate constant of translation, $k_{dProtein}$ represents a rate constant of decomposition of the protein, and $\mu$ represents the specific growth rate.

3. The method according to claim 1 or 2, wherein the growth rate factor is a function of the specific growth rate or a function of time.

4. The method according to any one of claims 1 to 3, wherein the specific growth rate is represented as a function of time, and the function is obtained by generating a mathematic equation from measurement data of the specific growth rate in the production process.

5. The method according to any one of claims 1 to 4, wherein the growth rate factor representing the formation rate is obtained by generating a mathematic equation expressing measurement data of the formation rate in the production process.

6. The method according to any one of claims 1 to 5, wherein the growth rate factor representing the inflow rate and/or the outflow rate is obtained by generating a mathematic equation expressing measurement data of the inflow rate and/or the outflow rate in the production process.

7. The method according to any one of claims 1 to 6, wherein the metabolite taken up into the cells is a substrate and/or an organic substance in a medium.

8. The method according to any one of claims 1 to 7, wherein the metabolite excreted out of the cells is an objective substance and/or a by-product.

9. The method according to claim 8, wherein the metabolite excreted out of the cells is an amino acid, an organic acid and/or carbon dioxide.

10. The method according to claim 9, wherein the metabolite excreted out of the cells is an amino acid or an organic acid.

11. The method according to any one of claims 1 to 10, wherein at least one of said parameters is a rate constant of transcription and/or a rate constant of translation.

12. The method according to any one of claims 1 to 11, wherein the cells are those of a microorganism having an amino acid producing ability and/or an organic acid producing ability.

13. The method according to claim 12, wherein the microorganism is *Escherichia coli.*

14. The method according to any one of claims 1 to 13, wherein a composition of the cells, are represented by a mathematical equation using the specific growth rate of the cells or the cells' equivalent index concerning the growth.

15. A computer program product for effecting a simulation of a substance-production process that uses cells, wherein said simulation is based on a set of differential equations that represent intracellular metabolites and gene expression, comprising:

   (a) computer code for including a specific growth rate of cells, expressed as a differential equation, in the set of differential equations;
   (b) computer code for assigning values for parameters in the set of differential equations, wherein at least one of said parameters is represented as a growth rate factor;
   (c) computer code for incorporating in the set of differential equations a formation rate for formation of a cell component from an intracellular metabolite, wherein said formation rate is represented as a growth rate factor;
   (d) computer code for incorporating in the set of differential equations an inflow rate of a metabolite taken up from the outside of the cells and/or an outflow rate of a metabolite excreted out of the cells from the inside of the cells, wherein said inflow rate and said outflow rate are represented, respectively, as a growth rate factor;
   (e) computer code for solving the set of differential equations; and
   (f) computer code for generating data representative of the substance-production process.

16. A system for effecting a simulation of a substance-production process that uses cells, wherein said simulation is based on a set of differential equations that represent intracellular metabolites and gene expression, comprising:

   a processor for processing information; and
   a storing means, including:

      (a) computer code for including a specific growth rate of cells, expressed as a differential equation, in the set of differential equations;
      (b) computer code for assigning values for parameters in the set of differential equations, wherein at least one of said parameters is represented as a growth rate factor;
      (c) computer code for incorporating in the set of differential equations a formation rate for formation of a cell component from an intracellular metabolite, wherein said formation rate is represented as a growth rate factor;
      (d) computer code for incorporating in the set of differential equations an inflow rate of a metabolite taken up from the outside of the cells and/or an outflow rate of a metabolite excreted out of the cells from the inside of the cells, wherein said inflow rate and said outflow rate are represented, respectively, as a growth rate factor;

(e) computer code for solving the set of differential equations; and
(f) computer code for generating data representative of the substance-production process.

Fig. 1

**(A)**

**(B)**

Fig. 2

Fig. 3

**(A)**

**(B)**

Fig. 4

EP 1 752 899 A1

```
                    ┌─────────┐
                    │  Start  │
                    └────┬────┘
                         │
        ┌────────────────┴────────────────┐
        │  Storing differential equations  │       S1
        └────────────────┬────────────────┘
                         │
        ┌────────────────┴────────────────┐
        │   Storing values of parameters   │       S2
        └────────────────┬────────────────┘
                         │
        ┌────────────────┴────────────────┐
        │      Calculating solutions of    │       S3
        │       differential equations     │
        └────────────────┬────────────────┘
                         │
                    ┌────┴────┐
                    │   End   │
                    └─────────┘
```

# Fig. 5

```
           3      ┌──────────────┐  1                  4
 ┌──────────────┐ │   Central    │    ┌──────────────┐
 │ Input portion ├─┤  processing  ├────┤ Output portion│
 └──────────────┘ │   portion    │    └──────────────┘
                  └──────┬───────┘  2
                         │
                  ┌──────┴───────┐
                  │Storing potion │
                  └──────────────┘
```

# Fig. 6

Fig. 7

Fig. 8A

Fig. 8B

Fig. 9A

Fig. 9B

Fig. 10A

Fig. 10B

EP 1 752 899 A1

Fig. 11A

EP 1 752 899 A1

Fig. 11B

Fig. 12A

Fig. 12B

EP 1 752 899 A1

European Patent
Office

**EUROPEAN SEARCH REPORT**

Application Number

EP 06 01 6619

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | VARNER JEFFREY D: "Large-scale prediction of phenotype: Concept" 20 September 2000 (2000-09-20), BIOTECHNOLOGY AND BIOENGINEERING, VOL. 69, NR. 6, PAGE(S) 664-678 , XP002412857 ISSN: 0006-3592 * the whole document * | 1,15,16 | INV. G06F19/00 |
| X,D | PRAMANIK J, KEASLING JD: "Stoichiometric Model of Escherichia coli Metabolism: Incorporation of Growth-Rate Depedent Biomass Composition and Mechanistic Energy Requirements" BIOTECHNOLOGY AND BIOENGINEERING, vol. 56, no. 4, 20 November 1997 (1997-11-20), pages 398-421, XP002412836 * the whole document * | 1,15,16 | |
| A,D | CHASSAGNOLE C ET AL: "Dynamic Modelling of the Central Carbon Metabolism of Escherichia coli" BIOTECHNOLOGY AND BIOENGINEERING, vol. 79, no. 1, 5 July 2002 (2002-07-05), pages 53-73, XP002412837 * the whole document * | 1,15,16 | TECHNICAL FIELDS SEARCHED (IPC) G06F |

The present search report has been drawn up for all claims

| Place of search | Date of completion of the search | Examiner |
|---|---|---|
| Munich | 22 December 2006 | Sisk, Aisling |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020022947 A
- WO 2004081862 A
- WO 0307217 A
- WO 0255995 A
- WO 0205205 A
- JP 2003180400 A
- WO 2005001736 A

**Non-patent literature cited in the description**

- **ISHII, N. et al.** *J. Biotechnol.,* 2004, vol. 113, 281-294
- **TEUSINK, B. et al.** *Eur. J. Biochem.,* 2000, vol. 267, 5313-5329
- **CHASSAGNOLE, C. et al.** *Biotechnol. Bioeng.,* 2002, vol. 26, 203-216
- **WANG, J. et al.** *J. Biotechnol.,* 2001, vol. 92, 133-158
- **SCHMID J.W. et al.** *Metab. Eng.,* 2004, vol. 6, 364-377
- **VARNER, J.D.** *Biotechnol. Bioeng.,* 2000, vol. 69, 664-678
- **JEONG et al.** *Biotechnol. Bioeng.,* 1990, vol. 35, 160-184
- **TOMITA et al.** *Bioinformatics,* 1999, vol. 15, 72-84
- **KANEHISA, M. et al.** *Nucleic Acids Res.,* 2004, vol. 32, 277-280
- **KESELER et al.** *Nucleic Acids Res.,* 2005, vol. 33, D334-D337
- **SEGEL I.H.** Enzyme Kinetics: Behavior and Analysis of Rapid Equilibrium and Steady-State Enzyme Systems. John Wiley & Sons, 1975
- **MENDES, P.** *Comput. Applic. Biosci.,* 1993, vol. 9, 563-571
- **SAURO, H.M.** *Comput. Appl. Biosci.,* 1993, vol. 9, 441-450
- **TOMITA, M. et al.** *Bioinformatics,* 1999, vol. 15, 72-84
- **BREMER ; DENNIS.** In Escherichia coli and Salmonella: Cellular and Molecular Biology. American Society for Microbiology Press, 1996, 1553-1569
- **SAVINELL J.M. ; PALSSON B.O.** *J. Theor. Biol.,* 1992, vol. 154, 421-454
- **VALLINO, J.J. ; STEPHANOPOULOS, G.** *Biotechnol. Bioeng.,* 1993, vol. 41, 633-646
- **PRAMANIK, J. ; KEASLING J.D.** *Biotechnol. Bioeng.,* 1997, vol. 56, 398-421
- **PRAMANIK, J. ; KEASLING J.D.** *Biotechnol. Bioeng.,* 1998, vol. 60, 230-238
- **ROHWER et al.** *J. Biol. Chem.,* 2000, vol. 275, 34909-34921
- **LEE ; BAILY.** *Biotech. Bioeng.,* 1984, vol. 26, 66-72
- **MILLER, C.G.** In Escherichia coli and Salmonella: Cellular and Molecular Biology. American Society for Microbiology Press, 1996, 680-691
- *Biotech. Bioeng.,* 1984, vol. 26, 66-72
- *Escherichia coli and Salmonella,* 1996, vol. 44, 680-691
- *Biotech Bioeng.,* 2002, vol. 79, 53-73
- *Biotech. Bioeng.,* 2002, vol. 79, 53-73
- *Metab. Eng.,* 2002, vol. 4, 182-192
- *Escherichia coli and Salmonella,* 1996, vol. 87, 1357-1381
- *Biochem. J.,* 2001, vol. 356, 433-444
- *Nature,* 1983, vol. 305, 286-290
- *Anal. Biochem.,* 2001, vol. 295, 129-137
- *Biomol. Eng.,* 2002, vol. 19, 5-15
- *Mol. Microbiol.,* 1993, vol. 10, 341-350
- **QUAIL ; GUEST.** *Mol. Microbiol.,* 1995, vol. 15, 519-529
- **CORTAY et al.** *EMBO J.,* 1991, vol. 10, 675-679
- **REDDY ; KAMIREDDI.** *J. Bacteriol.,* 1998, vol. 180, 732-736
- **CUNNINGHAM ; GUEST.** *Microbiology,* 1998, vol. 144, 2113-2123
- **CHUNG et al.** *J. Bacteriol.,* 1993, vol. 175, 4572-4575
- **BREMER ; DENNIS.** Escherichia coli and Salmonella: Cellular and Molecular Biology. American Society for Microbiology Press, 1996, 1553-1569
- **SELINGER et al.** *Genome Res.,* 2003, vol. 13, 216-223

- **CHASSAGNOLE et al.** *Biotechnol. Bioeng.,* 2002, vol. 79, 53-73
- **NEIDHARDT ; UMBARGER.** Escherichia coli and Salmonella: Cellular and Molecular Biology. American Society for Microbiology, 1996, 13-16
- **PRAMANIK ; KEASLING.** *Biotechnol. Bioeng.,* 1997, vol. 56, 398-421
- **STEPHANOPOULOS et al.** Metabolic Engineering: Principles and Methodologies. Academic Press, 1998